(19) Europäisches Patentamt
European Patent Office
Office européen des brevets

(11) EP 3 399 037 A1

(12) **EUROPEAN PATENT APPLICATION**
published in accordance with Art. 153(4) EPC

(43) Date of publication:
**07.11.2018 Bulletin 2018/45**

(21) Application number: **16881846.6**

(22) Date of filing: **29.12.2016**

(51) Int Cl.:
***C12N 15/113*** *(2010.01)* ***A61K 31/7088*** *(2006.01)*
***A61K 31/7105*** *(2006.01)* ***A61K 48/00*** *(2006.01)*
***A61P 27/02*** *(2006.01)*

(86) International application number:
**PCT/JP2016/089216**

(87) International publication number:
**WO 2017/115872 (06.07.2017 Gazette 2017/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.12.2015 JP 2015257713**

(71) Applicants:
- **National University Corporation Hokkaido University**
  **Sapporo-shi, Hokkaido 060-0808 (JP)**
- **Tokyo Medical University**
  **Shinjuku-ku, Tokyo 160-8402 (JP)**
- **Bonac Corporation**
  **Kurume-shi, Fukuoka 839-0861 (JP)**

(72) Inventors:
- **ISHIDA, Susumu**
  **Sapporo-shi**
  **Hokkaido 060-0808 (JP)**
- **KANDA, Atsuhiro**
  **Sapporo-shi**
  **Hokkaido 060-0808 (JP)**
- **KURODA, Masahiko**
  **Tokyo 160-8402 (JP)**
- **TOYOFUKU, Hidekazu**
  **Kurume-shi**
  **Fukuoka 839-0861 (JP)**

(74) Representative: **J A Kemp**
**14 South Square**
**Gray's Inn**
**London WC1R 5JJ (GB)**

(54) **SINGLE-STRANDED NUCLEIC ACID MOLECULE INHIBITING EXPRESSION OF PRORENIN GENE OR PRORENIN RECEPTOR GENE, AND USE THEREOF**

(57) The present invention provides a single-stranded nucleic acid molecule inhibiting expression of a prorenin gene or a prorenin receptor gene, comprising only region (X), linker region (Lx) and region (Xc), wherein the aforementioned linker region (Lx) has a non-nucleotide structure comprising at least one of a pyrrolidine skeleton and a piperidine skeleton, and at least one of the aforementioned region (X) and the aforementioned region (Xc) comprises an expression inhibitory sequence comprising a nucleotide sequence consisting of at least 18 continuous nucleotides in any of an expression inhibitory sequence of a prorenin gene shown in SEQ ID NO: 1 to 5 and an expression inhibitory sequence of prorenin receptor gene shown in SEQ ID NO: 6 to 11.

Fig. 1

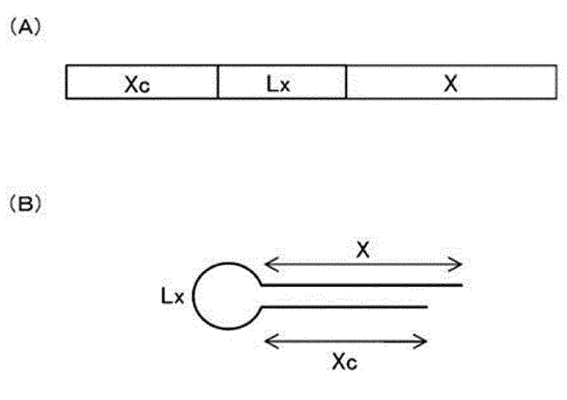


EP 3 399 037 A1

SEQUENCE LISTING

<110> NATIONAL UNIVERSITY CORPORATION HOKKAIDO UNIVERSITY
TOKYO MEDICAL UNIVERSITY
BONAC CORPORATION

<120> Single-stranded nucleic acid molecule for suppressing prorenin gene or prorenin receptor gene expression and use thereof

<130> 092542

<150> JP 2015-257713
<151> 2015-12-29

<160> 64

<170> PatentIn version 3.5

<210> 1
<211> 21
<212> RNA
<213> Artificial Sequence

<220>
<223> expression inhibitory sequence

<400> 1
ugauguuguc gaagauaggg g 21

<210> 2
<211> 21
<212> RNA
<213> Artificial Sequence

<220>
<223> expression inhibitory sequence

<400> 2
uugauauagu ggaaauuccc u 21

<210> 3
<211> 21
<212> RNA
<213> Artificial Sequence

<220>
<223> expression inhibitory sequence

<400> 3
ugauauagug gaaauucccu u 21

<210> 4
<211> 21
<212> RNA
<213> Artificial Sequence

<220>
<223> expression inhibitory sequence

<400> 4

uagaacuuuc ggaugaaggu g 21

<210> 5
<211> 21
<212> RNA
<213> Artificial Sequence

<220>
<223> expression inhibitory sequence

<400> 5
aucaaacucu guguagaacu u 21

<210> 6
<211> 21
<212> RNA
<213> Artificial Sequence

<220>
<223> expression inhibitory sequence

<400> 6
uuccauuucg gaaaacaaca g 21

<210> 7
<211> 21
<212> RNA
<213> Artificial Sequence

<220>
<223> expression inhibitory sequence

<400> 7
aauuuccauu ucggaaaaca a 21

<210> 8
<211> 21
<212> RNA
<213> Artificial Sequence

<220>
<223> expression inhibitory sequence

<400> 8
uuauaugcaa gguuauaggg a 21

<210> 9
<211> 21
<212> RNA
<213> Artificial Sequence

<220>
<223> expression inhibitory sequence

<400> 9
uauaugcaag guuauaggga c 21

<210>  10
<211>  21
<212>  RNA
<213>  Artificial Sequence

<220>
<223>  expression inhibitory sequence

<400>  10
aaaaggaacu gcauucucca a                                                21

<210>  11
<211>  21
<212>  RNA
<213>  Artificial Sequence

<220>
<223>  expression inhibitory sequence

<400>  11
uuaguaucca uauuagccca u                                                21

<210>  12
<211>  21
<212>  RNA
<213>  Artificial Sequence

<220>
<223>  complementary sequence to expression inhibitory sequence

<400>  12
ccuaucuucg acaacaucau c                                                21

<210>  13
<211>  21
<212>  RNA
<213>  Artificial Sequence

<220>
<223>  complementary sequence to expression inhibitory sequence

<400>  13
ggaauuucca cuauaucaac c                                                21

<210>  14
<211>  21
<212>  RNA
<213>  Artificial Sequence

<220>
<223>  complementary sequence to expression inhibitory sequence

<400>  14
gggaauuucc acuauaucaa c                                                21

<210>  15
<211>  21
<212>  RNA

<213> Artificial Sequence

<220>
<223> complementary sequence to expression inhibitory sequence

<400> 15
ccuucauccg aaaguucuac a 21

<210> 16
<211> 21
<212> RNA
<213> Artificial Sequence

<220>
<223> complementary sequence to expression inhibitory sequence

<400> 16
guucuacaca gaguuugauc g 21

<210> 17
<211> 21
<212> RNA
<213> Artificial Sequence

<220>
<223> complementary sequence to expression inhibitory sequence

<400> 17
guuguuuucc gaaauggaaa u 21

<210> 18
<211> 21
<212> RNA
<213> Artificial Sequence

<220>
<223> complementary sequence to expression inhibitory sequence

<400> 18
guuuuccgaa auggaaauug g 21

<210> 19
<211> 21
<212> RNA
<213> Artificial Sequence

<220>
<223> complementary sequence to expression inhibitory sequence

<400> 19
ccuauaaccu ugcauauaag u 21

<210> 20
<211> 21
<212> RNA
<213> Artificial Sequence

<220>

<223>  complementary sequence to expression inhibitory sequence

<400>  20
cccuauaacc uugcauauaa g                                                21

<210>  21
<211>  21
<212>  RNA
<213>  Artificial Sequence

<220>
<223>  complementary sequence to expression inhibitory sequence

<400>  21
ggagaaugca guuccuuuua g                                                21

<210>  22
<211>  21
<212>  RNA
<213>  Artificial Sequence

<220>
<223>  complementary sequence to expression inhibitory sequence

<400>  22
gggcuaauau ggauacuaaa a                                                21

<210>  23
<211>  48
<212>  RNA
<213>  Artificial Sequence

<220>
<223>  single-stranded nucleic acid molecule

<400>  23
ccuaucuucg acaacaucau cccgggauga uguugucgaa gauagggg                   48

<210>  24
<211>  48
<212>  RNA
<213>  Artificial Sequence

<220>
<223>  single-stranded nucleic acid molecule

<400>  24
ggaauuucca cuauaucaac cccggggguug auauagugga aauucccu                   48

<210>  25
<211>  48
<212>  RNA
<213>  Artificial Sequence

<220>
<223>  single-stranded nucleic acid molecule

<400>  25

gggaauuucc acuauaucaa cccggguuga uauaguggaa auucccuu 48

<210> 26
<211> 48
<212> RNA
<213> Artificial Sequence

<220>
<223> single-stranded nucleic acid molecule

<400> 26
ccuucauccg aaaguucuac accgguguag aacuuucgga ugaaggug 48

<210> 27
<211> 48
<212> RNA
<213> Artificial Sequence

<220>
<223> single-stranded nucleic acid molecule

<400> 27
guucuacaca gaguuugauc gccggcgauc aaacucugug uagaacuu 48

<210> 28
<211> 48
<212> RNA
<213> Artificial Sequence

<220>
<223> single-stranded nucleic acid molecule

<400> 28
guuguuuucc gaaauggaaa uccggauuuc cauuucggaa aacaacag 48

<210> 29
<211> 48
<212> RNA
<213> Artificial Sequence

<220>
<223> single-stranded nucleic acid molecule

<400> 29
guuuuccgaa auggaaauug gccggccaau uuccauuucg gaaaacaa 48

<210> 30
<211> 48
<212> RNA
<213> Artificial Sequence

<220>
<223> single-stranded nucleic acid molecule

<400> 30
ccuauaaccu ugcauauaag uccggacuua uaugcaaggu uauaggga 48

<210> 31
<211> 48
<212> RNA
<213> Artificial Sequence

<220>
<223> single-stranded nucleic acid molecule

<400> 31
cccuauaacc uugcauauaa gccggcuuau augcaagguu auagggac 48

<210> 32
<211> 48
<212> RNA
<213> Artificial Sequence

<220>
<223> single-stranded nucleic acid molecule

<400> 32
ggagaaugca guuccuuuua gccggcuaaa aggaacugca uucuccaa 48

<210> 33
<211> 48
<212> RNA
<213> Artificial Sequence

<220>
<223> single-stranded nucleic acid molecule

<400> 33
gggcuaauau ggauacuaaa accgguuuua guauccauau uagcccau 48

<210> 34
<211> 20
<212> DNA
<213> Artificial Sequence

<220>
<223> primer

<400> 34
gctttctcag ccaggacatc 20

<210> 35
<211> 20
<212> DNA
<213> Artificial Sequence

<220>
<223> primer

<400> 35
tgggagatga tgttgtcgaa 20

<210> 36
<211> 20
<212> DNA

<213>  Artificial Sequence

<220>
<223>  primer

<400>  36
tcgtaccctt tggagaatgc                                                    20

<210>  37
<211>  20
<212>  DNA
<213>  Artificial Sequence

<220>
<223>  primer

<400>  37
gagccaactg caaaacaaca                                                    20

<210>  38
<211>  23
<212>  DNA
<213>  Artificial Sequence

<220>
<223>  primer

<400>  38
ggagaaggct ggggctcatt tgc                                                23

<210>  39
<211>  23
<212>  DNA
<213>  Artificial Sequence

<220>
<223>  primer

<400>  39
tggccagggg tgctaagcag ttg                                                23

<210>  40
<211>  20
<212>  DNA
<213>  Artificial Sequence

<220>
<223>  primer

<400>  40
aggcagtgtc atttcgtacc                                                    20

<210>  41
<211>  22
<212>  DNA
<213>  Artificial Sequence

<220>

<223> primer

<400> 41
gccttcccta ccatatacac tc 22

<210> 42
<211> 20
<212> DNA
<213> Artificial Sequence

<220>
<223> primer

<400> 42
accccacgaa gtgttggata 20

<210> 43
<211> 20
<212> DNA
<213> Artificial Sequence

<220>
<223> primer

<400> 43
aagcagatgg ccacagaact 20

<210> 44
<211> 21
<212> DNA
<213> Artificial Sequence

<220>
<223> primer

<400> 44
tggtcgttct cctgttcttt c 21

<210> 45
<211> 21
<212> DNA
<213> Artificial Sequence

<220>
<223> primer

<400> 45
accctggcga tcttaatatg c 21

<210> 46
<211> 20
<212> DNA
<213> Artificial Sequence

<220>
<223> primer

<400> 46

caaactttgc tttccctggt 20

<210> 47
<211> 20
<212> DNA
<213> Artificial Sequence

<220>
<223> primer

<400> 47
caagggcata tccaacaaca 20

<210> 48
<211> 20
<212> DNA
<213> Artificial Sequence

<220>
<223> primer

<400> 48
tgtgggcaac ctattccacc 20

<210> 49
<211> 20
<212> DNA
<213> Artificial Sequence

<220>
<223> primer

<400> 49
agctgacgca atgtgactga 20

<210> 50
<211> 20
<212> DNA
<213> Artificial Sequence

<220>
<223> primer

<400> 50
gcagactttg ctttccttgg 20

<210> 51
<211> 20
<212> DNA
<213> Artificial Sequence

<220>
<223> primer

<400> 51
tccactttcg ctgatgacac 20

<210> 52
<211> 18
<212> DNA
<213> Artificial Sequence

<220>
<223> primer

<400> 52
ttggctcagc cagatgca 18

<210> 53
<211> 23
<212> DNA
<213> Artificial Sequence

<220>
<223> primer

<400> 53
cctactcatt gggatcatct tgc 23

<210> 54
<211> 20
<212> DNA
<213> Artificial Sequence

<220>
<223> primer

<400> 54
cctgtttcct ggctctgaag 20

<210> 55
<211> 20
<212> DNA
<213> Artificial Sequence

<220>
<223> primer

<400> 55
gtctgctgag acccctcttg 20

<210> 56
<211> 23
<212> DNA
<213> Artificial Sequence

<220>
<223> primer

<400> 56
cacagaggat accactccca aca 23

<210> 57
<211> 22
<212> DNA

<213>  Artificial Sequence

<220>
<223>  primer

<400>  57
tccacgattt cccagagaaa ca                                                22

<210>  58
<211>  22
<212>  DNA
<213>  Artificial Sequence

<220>
<223>  primer

<400>  58
ggtgcctatg tctcagcctc tt                                                22

<210>  59
<211>  21
<212>  DNA
<213>  Artificial Sequence

<220>
<223>  primer

<400>  59
cgatcacccc gaagttcagt a                                                 21

<210>  60
<211>  21
<212>  DNA
<213>  Artificial Sequence

<220>
<223>  primer

<400>  60
aggtcggtgt gaacggattt g                                                 21

<210>  61
<211>  23
<212>  DNA
<213>  Artificial Sequence

<220>
<223>  primer

<400>  61
tgtagaccat gtagttgagg tca                                               23

<210>  62
<211>  48
<212>  RNA
<213>  Artificial Sequence

<220>

```
<223>  single-stranded nucleic acid molecule

<400>  62
uacuauucga cacgcgaagu uccggaacuu cgcgugucga auaguauu                  48


<210>  63
<211>  19
<212>  DNA
<213>  Artificial Sequence

<220>
<223>  primer

<400>  63
ctggtggcgg gtgctttag                                                  19


<210>  64
<211>  21
<212>  DNA
<213>  Artificial Sequence

<220>
<223>  primer

<400>  64
gctacgtctg ggattcgatc t                                               21
```

## Description

[Technical Field]

The present invention relates to a nucleic acid molecule that inhibits expression of prorenin gene or prorenin receptor gene, a composition containing same and use thereof.

[Background Art]

Renin-angiotensin system (RAS) is mainly involved in the control of blood pressure and maintenance of electrolyte balance (circulatory RAS), and plays a role of cell differentiation and proliferation, tissue repair and the like in the local parts of organs (tissue RAS). In diabetic retinopathy and choroidal neovascularization, activation of tissue RAS is involved in the blood vessel pathology since it stimulates signal pathway via angiotensin type 1 receptor, and induces expression of inflammation-related molecules such as VEGF, MCP-1, ICAM-1 and the like.

Prorenin, which is considered to be merely an inactive precursor of renin, binds to a common transmembrane type receptor for renin and prorenin (ATP6AP2; ATPase, H+ transporting, lysosomal accessory protein 2, also described as "prorenin receptor" in the present specification), thereby acquiring the ability to bind to angiotensinogen, causes activation of tissue RAS, simultaneously causes activation of ERK as an intracellular signal mediated by the prorenin receptor, thereby also inducing VEGF and MCP-1. These tissue RAS activation and RAS-independent intracellular signaling activation by the prorenin-prorenin receptor are collectively referred to as receptor-associated prorenin system (RAPS).

As mentioned above, RAPS is known to be deeply involved in the inflammation and angiogenesis of ocular tissue through two different actions, and intervention in RAPS is considered to have an important meaning as a therapeutic strategy of eye diseases accompanied by inflammation or angiogenesis.

As a technique for inhibiting gene expression, for example, RNA interference (RNAi) is known. Inhibition of gene expression by RNA interference is generally carried out, for example, by administering a short double-stranded RNA molecule to a cell or the like. The aforementioned double-stranded RNA molecule is generally called siRNA (small interfering RNA). While studies of various genes by using siRNA have been conducted, they are associated with problems of possible blood instability and side effects due to immune response and the like, and provision of a more effective nucleic acid molecule has been desired.

In recent years, the present inventor has newly developed a more effective single-stranded nucleic acid molecule replacing siRNA (patent documents 1, 2, non-patent document 1).

[Document List]

[Patent documents]

patent document 1: WO 2012/005368
patent document 2: WO 2012/017919

[non-patent document]

non-patent document 1: Hamasaki et al., PLoS ONE, 7(8), e42655, doi:10.1371, 2012

[SUMMARY OF THE INVENTION]

[Problems to be Solved by the Invention]

The present invention aims to provide a nucleic acid molecule effective for the treatment of diseases involving expression of prorenin gene or prorenin receptor gene, and a medicament using same.

[Means of Solving the Problems]

The present inventors have found that a single-stranded nucleic acid molecule in which a region containing a nucleotide sequence targeting a particular partial sequence within the prorenin gene or prorenin receptor gene and a region containing a complementary chain sequence thereof are linked using a particular linker markedly inhibits expression of a prorenin gene or a prorenin receptor gene and also improve pathology in a uveitis animal model, which resulted in the completion of the present invention.

Accordingly, the present invention is as shown below.

[1] A single-stranded nucleic acid molecule inhibiting expression of a prorenin gene or a prorenin receptor gene, comprising only region (X), linker region (Lx) and region (Xc), wherein the aforementioned linker region (Lx) has a non-nucleotide structure comprising at least one of a pyrrolidine skeleton and a piperidine skeleton, and
one of the aforementioned region (X) and the aforementioned region (Xc) comprises an expression inhibitory sequence comprising a nucleotide sequence consisting of at least 18 continuous nucleotides in any nucleotide sequence selected from a sequence complementary to a part of a prorenin gene represented by the following SEQ ID NO: 1 to 5:

(SEQ ID NO: 1) 5'-UGAUGUUGUCGAAGAUAGGGG-3'
(SEQ ID NO: 2) 5'-UUGAUAUAGUGGAAAUUCCCU-3'
(SEQ ID NO: 3) 5'-UGAUAUAGUGGAAAUUCCCUU-3'
(SEQ ID NO: 4) 5'-UAGAACUUUCGGAUGAAGGUG-3'
(SEQ ID NO: 5) 5'-AUCAAACUCUGUGUAGAACUU-3'

and a sequence complementary to a part of a prorenin receptor gene represented by the following SEQ ID NO: 6 to 11:

(SEQ ID NO: 6) 5'-UUCCAUUUCGGAAAACAACAG-3'
(SEQ ID NO: 7) 5'-AAUUUCCAUUUCGGAAAACAA-3'
(SEQ ID NO: 8) 5'-UUAUAUGCAAGGUUAUAGGGA-3'
(SEQ ID NO: 9) 5'-UAUAUGCAAGGUUAUAGGGAC-3'
(SEQ ID NO: 10) 5'-AAAAGGAACUGCAUUCUCCAA-3'
(SEQ ID NO: 11) 5'-UUAGUAUCCAUAUUAGCCCAU-3', and

the other comprises a nucleotide sequence complementary to the expression inhibitory sequence.
[2] The nucleic acid molecule of the above-mentioned [1], wherein the region (X), the linker region (Lx) and the region (Xc) are disposed in this order from the 3' -side to the 5'-side, and a base number (X) of the aforementioned region (X) and a base number (Xc) of the aforementioned region (Xc) satisfy the conditions of the following formula (1) or the formula (2):

$$X>Xc \ldots \quad (1)$$

$$X=Xc \ldots \quad (2).$$

[3] The nucleic acid molecule of the above-mentioned [2], wherein the base number (X) of the aforementioned region (X) and the base number (Xc) of the aforementioned region (Xc) satisfy the conditions of the following formula (3):

$$X-Xc=1,\ 2 \text{ or } 3 \ldots \quad (3).$$

[4] The nucleic acid molecule of any of the above-mentioned [1] to [3], wherein the base number (Xc) of the aforementioned region (Xc) is 19 - 30.
[5] The nucleic acid molecule of any of the above-mentioned [1] to [4], wherein the aforementioned linker region (Lx) is represented by the following formula (I):

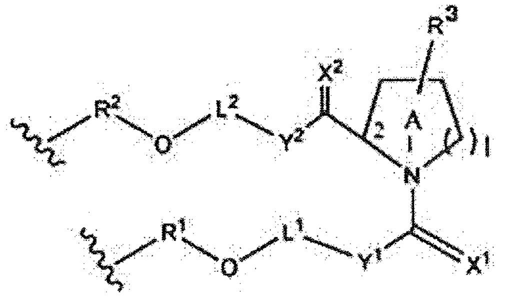

・・・(I)

wherein,

$X^1$ and $X^2$ are each independently $H_2$, O, S, or NH;
$Y^1$ and $Y^2$ are each independently a single bond, $CH_2$, NH, O, or S;
$R^3$ is a hydrogen atom or a substituent bonded to C-3, C-4, C-5 or C-6 on ring A,
$L^1$ is an alkylene chain composed of n atoms, and a hydrogen atom on an alkylene carbon atom may or may not be substituted with OH, $OR^a$, $NH_2$, $NHR^a$, $NR^aR^b$, SH, or $SR^a$, or
$L^1$ is a polyether chain obtained by substituting at least one carbon atom on the aforementioned alkylene chain with an oxygen atom,
provided that: when $Y^1$ is NH, O, or S, an atom bound to $Y^1$ in $L^1$ is carbon, an atom bound to $OR^1$ in $L^1$ is carbon, and oxygen atoms are not adjacent to each other;
$L^2$ is an alkylene chain composed of m atoms, and a hydrogen atom on an alkylene carbon atom may or may not be substituted with OH, $OR^c$, $NH_2$, $NHR^c$, $NR^cR^d$, SH, or $SR^c$, or
$L^2$ is a polyether chain obtained by substituting at least one carbon atom on the aforementioned alkylene chain with an oxygen atom,
provided that: when $Y^2$ is NH, O, or S, an atom bound to $Y^2$ in $L^2$ is carbon, an atom bound to $OR^2$ in $L^2$ is carbon, and oxygen atoms are not adjacent to each other;
$R^a$, $R^b$, $R^c$, and $R^d$ are each independently a substituent or a protecting group; 1 is 1 or 2;
m is an integer in the range from 0 to 30;
n is an integer in the range from 0 to 30;
in ring A, one carbon atom other than C-2 on the aforementioned ring A may be substituted by nitrogen, oxygen or sulfur,
the aforementioned ring A may contain a carbon-carbon double bond or a carbon-nitrogen double bond,
the aforementioned regions (Xc) and (X) are each linked to the aforementioned linker region (Lx) via $-OR^1-$ or $-OR^2-$,
wherein $R^1$ and $R^2$ may or may not be present, and when they are present, $R^1$ and $R^2$ are each independently a nucleotide residue or the aforementioned structure (I).

[6] The nucleic acid molecule of any of the above-mentioned [1] to [5], wherein the aforementioned linker region (Lx) is represented by the following formula (I-4a) or (I-6a):

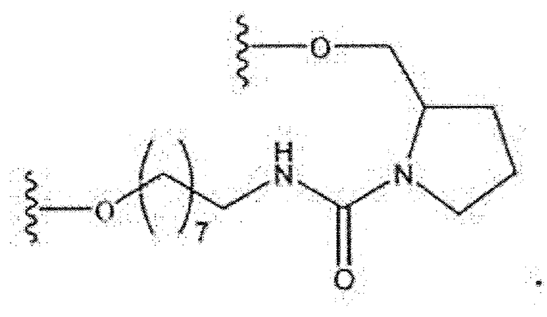

・・・(I-4a)

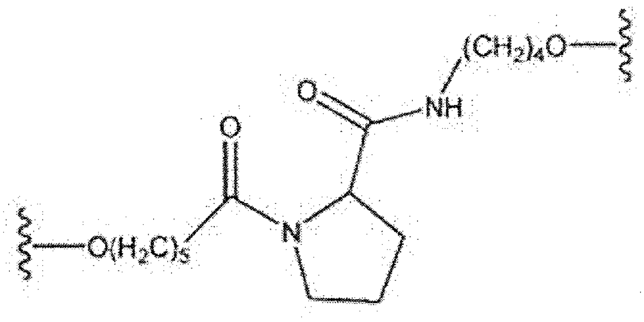

... (I-6a)

[7] The nucleic acid molecule of any of the above-mentioned [1] to [6], which is an RNA molecule.
[8] The nucleic acid molecule of any of the above-mentioned [1] to [7], comprising at least one modified residue.
[9] The nucleic acid molecule of any of the above-mentioned [1] to [8], comprising a labeling substance.
[10] The nucleic acid molecule of any of the above-mentioned [1] to [9], comprising a stable isotope.
[11] The nucleic acid molecule of any of the above-mentioned [1] to [10], wherein a total of the base number is not less than 38.
[12] The nucleic acid molecule of any of the above-mentioned [1] to [11], composed of a base sequence represented by any of the following SEQ ID NO: 23 to 33:

(SEQ ID NO: 23)
5'-CCUAUCUUCGACAACAUCAUCCC-Lx-GGGAUGAUGUUGUCGAAGAUAGGGG-3'
(SEQ ID NO: 24)
5'-GGAAUUUCCACUAUAUCAACCCC-Lx-GGGGUUGAUAUAGUGGAAAUUCCCU-3'
(SEQ ID NO: 25)
5'-GGGAAUUUCCACUAUAUCAACCC-Lx-GGGUUGAUAUAGUGGAAAUUCCCUU-3'
(SEQ ID NO: 26)
5'-CCUUCAUCCGAAAGUUCUACACC-Lx-GGUGUAGAACUUUCGGAUGAAGGUG-3'
(SEQ ID NO: 27)
5'- GUUCUACACAGAGUUUGAUCGCC-Lx-GGCGAUCAAACUCUGUGUAGAACUU-3'
(SEQ ID NO: 28)
5'-GUUGUUUUCCGAAAUGGAAAUCC-Lx-GGAUUUCCAUUUCGGAAAACAACAG-3'
(SEQ ID NO: 29)
5'-GUUUUCCGAAAUGGAAAUUGGCC-Lx-GGCCAAUUUCCAUUUCGGAAAACAA-3'
(SEQ ID NO: 30)
5'-CCUAUAACCUUGCAUAUAAGUCC-Lx-GGACUUAUAUGCAAGGUUAUAGGGA-3'
(SEQ ID NO: 31)
5'-CCCUAUAACCUUGCAUAUAAGCC-Lx-GGCUUAUAUGCAAGGUUAUAGGGAC-3'
(SEQ ID NO: 32)
5'-GGAGAAUGCAGUUCCUUUUAGCC-Lx-GGCUAAAAGGAACUGCAUUCUCCAA-3'
(SEQ ID NO: 33)
5'-GGGCUAAUAUGGAUACUAAAACC-Lx-GGUUUUAGUAUCCAUAUUAGCCCAU-3'

[13] An inhibitor of expression of a prorenin gene or a prorenin receptor gene, comprising the nucleic acid molecule of any of the above-mentioned [1] to [12].
[14] A medicament comprising the nucleic acid molecule of any of the above-mentioned [1] to [12].
[15] A therapeutic agent for an eye disease, comprising the nucleic acid molecule of any of the above-mentioned [1] to [12].
[16] The agent of the above-mentioned [15], wherein the eye disease is selected from the group consisting of diabetic retinopathy, uveitis and age-related macular degeneration.
[17] The agent of the above-mentioned [15], wherein the eye disease is selected from the group consisting of diabetic retinopathy, uveitis, age-related macular degeneration and glaucoma.
[18] A method for inhibiting expression of a prorenin gene or a prorenin receptor gene, comprising using the nucleic acid molecule of any of the above-mentioned [1] to [12].
[19] The method of the above-mentioned [18], comprising a step of administering the aforementioned nucleic acid molecule to a cell, a tissue or an organ.
[20] The method of the above-mentioned [19], wherein the aforementioned administration is performed in vitro.

[21] The nucleic acid molecule of any of the above-mentioned [1] to [12] for use in inhibiting expression of a prorenin gene or a prorenin receptor gene.
[22] Use of the nucleic acid molecule of any of the above-mentioned [1] to [12] for the production of an inhibitor of expression of a prorenin gene or a prorenin receptor gene.

[Effect of the Invention]

According to the nucleic acid molecule of the present invention, expression of a prorenin gene or a prorenin receptor gene can be remarkably inhibited in vivo. Therefore, the nucleic acid molecule can be a promising candidate of a therapeutic drug for a disease involving a receptor-associated prorenin system (RAPS), for example, eye diseases such as diabetic retinopathy, uveitis, age-related macular degeneration and the like. In addition, the molecule can be a promising candidate of a therapeutic drug for eye diseases such as diabetic retinopathy, uveitis, age-related macular degeneration, glaucoma and the like.

[Brief Description of the Drawings]

Fig. 1 shows schematic views illustrating an example of the nucleic acid molecule of the present invention.
Fig. 2 is a graph showing the relative value of the expression level of a prorenin gene in the Examples of the present invention.
Fig. 3 is a graph showing the relative value of the expression level of a prorenin receptor gene in the Examples of the present invention.
Fig. 4 is a graph showing the relative value of the expression level of a human prorenin receptor gene in the Examples of the present invention.
Fig. 5 is a graph showing the relative value of the expression level of a mouse prorenin receptor gene in the Examples of the present invention.
Fig. 6 is a graph showing the relative value of the expression level of a rat prorenin receptor gene in the Examples of the present invention.
Fig. 7 is a graph showing the relative value of the expression level of a human prorenin gene in the Examples of the present invention.
Fig. 8 is a graph showing the relative value of the expression levels of CCL2/MCP-1, ICAM-1, IL-6, TNF-$\alpha$ genes in the Examples of the present invention.
Fig. 9 shows nuclease resistance of the single-stranded nucleic acid molecule (A) and expression inhibitory effect on the prorenin receptor protein by the single-stranded nucleic acid molecule (B) in the Examples of the present invention.
Fig. 10 shows microscopic photographs of vitreous body (A-D, F, G) and graphs showing the number of leucocytes (E, H) in the Examples of the present invention.
Fig. 11 is a graph showing the relative value of the expression levels of inflammatory cytokine genes in the Examples of the present invention.
Fig. 12 shows microscopic photographs of retina (A-C), graph showing relative value of the length of photoreceptor outer segment (D), and immunoblot analysis results of rhodopsin (E) in the Examples of the present invention.
Fig. 13 shows microscopic photographs of vitreous body (A-D), a graph showing the number of leucocytes (E) and graphs showing the relative value of the expression levels of inflammatory cytokine genes (F-I) in the Examples of the present invention.
Fig. 14 shows a graph showing the area measurement results of CNV (A) and microscopic photographs of CNV (B-D) in the Examples of the present invention.

[Description of Embodiments]

Unless otherwise specified, the terms used in the present specification mean what is generally meant by them in the art.

1. Nucleic acid molecule for inhibiting expression of prorenin gene or prorenin receptor gene

(1) Expression inhibitory sequence and complementary sequence

The nucleic acid molecule of the present invention is, as mentioned above, a single-stranded nucleic acid

molecule inhibiting expression of a prorenin gene or a prorenin receptor gene, and characterized by comprising an expression inhibitory sequence of a prorenin gene or a prorenin receptor gene comprising a nucleotide sequence consisting of at least 18 continuous nucleotides in any nucleotide sequence selected from a sequence complementary to a part of a prorenin gene represented by the following SEQ ID NO: 1 to 5 and a sequence complementary to a part of a prorenin receptor gene represented by the following SEQ ID NO: 6 to 11 (to be referred to as "r nucleotide sequence"):

(SEQ ID NO: 1) 5'-UGAUGUUGUCGAAGAUAGGGG-3'
(SEQ ID NO: 2) 5'-UUGAUAUAGUGGAAAUUCCCU-3'
(SEQ ID NO: 3) 5'-UGAUAUAGUGGAAAUUCCCUU-3'
(SEQ ID NO: 4) 5'-UAGAACUUUCGGAUGAAGGUG-3'
(SEQ ID NO: 5) 5'-AUCAAACUCUGUGUAGAACUU-3'
(SEQ ID NO: 6) 5'-UUCCAUUUCGGAAAACAACAG-3'
(SEQ ID NO: 7) 5'-AAUUUCCAUUUCGGAAAACAA-3'
(SEQ ID NO: 8) 5'-UUAUAUGCAAGGUUAUAGGGA-3'
(SEQ ID NO: 9) 5'-UAUAUGCAAGGUUAUAGGGAC-3'
(SEQ ID NO: 10) 5'-AAAAGGAACUGCAUUCUCCAA-3'
(SEQ ID NO: 11) 5'-UUAGUAUCCAUAUUAGCCCAU-3'

The aforementioned expression inhibitory sequence may be, for example, a sequence composed of the aforementioned r nucleotide sequence, or a sequence containing the aforementioned r nucleotide sequence. When the aforementioned expression inhibitory sequence is a sequence containing a r nucleotide sequence, one or more nucleotides are added to the 5'-terminus and/or 3'-terminus of the r nucleotide sequence. In this case, as an expression inhibitory sequence as a whole is complementary to the prorenin gene or prorenin receptor gene to be the target (here, "complementary" is as defined for the below-mentioned complementary sequence). On the other hand, as mentioned below, the nucleotide (sequence) other than the expression inhibitory sequence in the region (X or Xc) containing the expression inhibitory sequence in the nucleic acid molecule of the present invention is not required to be complementary to the target gene.

The length of the aforementioned expression inhibitory sequence is not particularly limited and is, for example, 18 to 32 nucleotide length, preferably 19 to 30 nucleotide length, more preferably 19, 20, 21 nucleotide length. In the present invention, for example, the numerical value range of the base number discloses all positive integers belonging to the range. For example, an indication of "1 to 4 bases" means disclosure of any of "1, 2, 3, 4 bases" (hereinafter the same).

The single-stranded nucleic acid molecule of the present invention further contains a complementary sequence annealable with the aforementioned expression inhibitory sequence.

The aforementioned complementary sequence is not necessarily completely complementary as long as it can be annealed to the aforementioned expression inhibitory sequence under physiological conditions in the target cell. That is, the aforementioned complementary sequence may be a sequence having 100% complementarity in the region overlapping with the aforementioned expression inhibitory sequence. Alternatively, it may have complementary of, for example, 90% - 100%, 93% - 100%, 95% - 100%, 98% - 100%, 99% - 100% or the like.

When the aforementioned expression inhibitory sequence has a r nucleotide sequence in the nucleotide sequence shown in SEQ ID NO: n (n=1-11), the aforementioned complementary sequences include sequences complementary to r nucleotide sequences in the nucleotide sequences shown in the following SEQ ID NO: n+11 (to be referred to as "s nucleotide sequence"):

(SEQ ID NO: 12) 5'-CCUAUCUUCGACAACAUCAUC-3'
(SEQ ID NO: 13) 5'-GGAAUUUCCACUAUAUCAACC-3'
(SEQ ID NO: 14) 5'-GGGAAUUUCCACUAUAUCAAC-3'
(SEQ ID NO: 15) 5'-CCUUCAUCCGAAAGUUCUACA-3'
(SEQ ID NO: 16) 5'-GUUCUACACAGAGUUUGAUCG-3'
(SEQ ID NO: 17) 5'-GUUGUUUUCCGAAAUGGAAAU-3'
(SEQ ID NO: 18) 5'-GUUUUCCGAAAUGGAAAUUGG-3'
(SEQ ID NO: 19) 5'-CCUAUAACCUUGCAUAUAAGU-3'
(SEQ ID NO: 20) 5'-CCCUAUAACCUUGCAUAUAAG-3'
(SEQ ID NO: 21) 5'-GGAGAAUGCAGUUCCUUUUAG-3'
(SEQ ID NO: 22) 5'-GGGCUAAUAUGGAUACUAAAA-3'

The aforementioned complementary sequence may be, for example, a sequence composed of the aforementioned s nucleotide sequence, or a sequence containing the aforementioned s nucleotide sequence.

The length of the aforementioned complementary sequence is not particularly limited and is, for example, 18 - 32 nucleotide length, preferably 19 - 30 nucleotide length, more preferably 19, 20, 21 nucleotide length.
The aforementioned expression inhibitory sequence and the aforementioned complementary sequence may each be, for example, an RNA molecule consisting of ribonucleotide residues alone, or an RNA molecule containing a deoxyribonucleotide residue besides the ribonucleotide residues. When the uracil (U) residue is substituted by a deoxyribonucleotide residue, it is optionally substituted by either dT or dU.

(2) Single-stranded nucleic acid molecule

In the nucleic acid molecule of the present invention, a region containing the aforementioned expression inhibitory sequence and a region containing the aforementioned complementary sequence are indirectly linked via a linker region. The order of linkage of the region containing aforementioned expression inhibitory sequence and the region containing the aforementioned complementary sequence is not particularly limited and, for example, the 5'-terminus side of the aforementioned expression inhibitory sequence and the 3'-terminus side of the aforementioned complementary sequence may be linked via a linker region or the 3'-terminus side of the aforementioned expression inhibitory sequence and the 5'-terminus side of the aforementioned complementary sequence may be linked via a linker region. Preferred is the former.
The aforementioned linker region may be constituted of, for example, nucleotide residues, may be constituted of non-nucleotide residues, or may be constituted of both the nucleotide residues and non-nucleotide residues. Preferably, the aforementioned linker region is constituted of the non-nucleotide residue.
While specific examples of the single-stranded nucleic acid molecule of the present invention are shown below, the present invention is not limited thereto. (hairpin type single-stranded nucleic acid molecule) In preferable one embodiment, the single-stranded nucleic acid molecule of the present invention is a molecule wherein a 5'-side region and a 3'-side region are mutually annealed to form a double-stranded structure (stem structure). This can also be said a form of shRNA (small hairpin RNA or short hairpin RNA). shRNA has a hairpin structure and generally has one stem region and one loop region.
The nucleic acid molecule in this form comprises only region (X), linker region (Lx) and region (Xc), and has a structure wherein the aforementioned region (X) and the aforementioned region (Xc) having a complementary structure are linked via the aforementioned linker region (Lx). Specifically, one of the aforementioned region (X) and the aforementioned region (Xc) contains the aforementioned expression inhibitory sequence, and the other contains the aforementioned complementary sequence. Therefore, they can form a stem structure between the aforementioned region (X) and the aforementioned region (Xc) by intramolecular annealing, and the aforementioned linker region (Lx) becomes a loop structure.
The aforementioned nucleic acid molecule may have the aforementioned region (Xc), the aforementioned linker region (Lx) and the aforementioned region (X) from the 5'-side to the 3'-side in this order, or from the 3'-side to the 5'-side in this order. Preferred is the former. While the aforementioned expression inhibitory sequence may be disposed in any of the aforementioned region (X) and the aforementioned region (Xc), it is preferably disposed in the downstream side of the aforementioned complementary sequence, that is, in the 3'-side than the aforementioned complementary sequence. Therefore, when the aforementioned nucleic acid molecule has the aforementioned region (Xc), the aforementioned linker region (Lx) and the aforementioned region (X) from the 5'-side to the 3'-side in this order, the aforementioned expression inhibitory sequence is preferably disposed in the aforementioned region (X) .
One embodiment of the nucleic acid molecule in this form is shown in the schematic drawing of Fig. 1. Fig. 1(A) is a schematic drawing of an outline of the order of each region, Fig. 1(B) is a schematic drawing showing that the aforementioned nucleic acid molecule forms a double strand in the aforementioned molecule. As shown in Fig. 1(B), the aforementioned nucleic acid molecule forms a double strand between the aforementioned region (Xc) and the aforementioned region (X), and the aforementioned Lx region has a loop structure according to the length thereof. Fig. 1 merely shows the order of the aforementioned regions and the positional relationship of each region forming a double strand and, for example, the length of each region, the shape of the aforementioned linker region (Lx) and the like are not limited to these.
In the aforementioned nucleic acid molecule, the number of nucleotides in the aforementioned region (Xc) and the aforementioned region (X) is not particularly limited. While examples of the length of each region are shown below, the present invention is not limited thereto.
The lower limit of the umber of nucleotides in the aforementioned region (X) is, for example, 19, preferably 20. The upper limit thereof is, for example, 50, preferably 30, more preferably 25. Specific examples of the number of nucleotides in the aforementioned region (X) is 19 - 50, preferably 19 - 30, more preferably 19 - 25.
The lower limit of the number of nucleotides in the aforementioned region (Xc) is, for example, 19, preferably 20. The upper limit thereof is, for example, 50, preferably 30, more preferably 25. Specific examples of the number of nucleotides in the aforementioned region (Xc) is 19 - 50, preferably 19 - 30, more preferably 19 - 25.

The region containing the aforementioned expression inhibitory sequence (X or Xc) may be constituted of the aforementioned expression inhibitory sequence alone or may contain the aforementioned expression inhibitory sequence. The number of nucleotides in the aforementioned expression inhibitory sequence is as mentioned above. A region containing the aforementioned expression inhibitory sequence may further have an additional sequence at the 5'-side and/or 3'-side of the aforementioned expression inhibitory sequence. The additional sequence is preferably added to the aforementioned linker region (Lx) side. As described above, when the nucleic acid molecule of the present invention has the aforementioned region (Xc), the aforementioned linker region (Lx) and the aforementioned region (X) in this order from the 5'-side to the 3'-side, the aforementioned expression inhibitory sequence is preferably disposed in the aforementioned region (X) and, in this case, an additional sequence is added to the 5'-side of the aforementioned expression inhibitory sequence. The number of nucleotides in the aforementioned additional sequence is, for example, 1 - 31 nucleotides, preferably 1 - 21 nucleotides, more preferably 1 - 11 nucleotides, particularly preferably 1, 2, 3, 4, 5 or 6 nucleotides.

When the region containing the aforementioned expression inhibitory sequence (one of X and Xc) has an additional sequence on the aforementioned linker region (Lx) side, the region containing the aforementioned complementary sequence (the other of X and Xc) also contains a sequence complementary to the additional sequence on the aforementioned linker region (Lx) side.

The relationship between the number of nucleotides (X) in the aforementioned region (X) and the number of nucleotides (Xc) in the aforementioned region (Xc) satisfies, for example, the conditions of the following (1) or (2). In the former case, specifically, it satisfies, for example, the conditions of the following (4). For example, the nucleic acid molecule schematically shown in Fig. 1(B) satisfies the conditions of the following (1):

$$X>Xc... \quad (1)$$

X-Xc=1 - 10, preferably 1, 2 or 3,
more preferably 1 or 2... (4)

$$X=Xc... \quad (2)$$

The aforementioned linker region (Lx) is preferably a structure free of self-annealing in the region of itself.

When the aforementioned region (Lx) contains a nucleotide residue as mentioned above, the length thereof is not particularly limited. The aforementioned linker region (Lx) preferably has a length, for example, permitting the aforementioned region (X) and the aforementioned region (Xc) to form a double strand. The lower limit of the number of nucleotides in the aforementioned linker region (Lx) is, for example, 1, preferably 2, more preferably 3, and the upper limit thereof is, for example, 100, preferably 80, more preferably 50.

The full length of the aforementioned nucleic acid molecule is not particularly limited. In the aforementioned nucleic acid molecule, the lower limit of the total of the aforementioned number of nucleotides (number of nucleotides of full length) when the aforementioned linker region (Lx) contains a nucleotide residue is, for example, 38, preferably 42, more preferably 50, further preferably 51, particularly preferably 52, and the upper limit thereof is, for example, 300, preferably 200, more preferably 150, further preferably 100, particularly preferably 80.

In the aforementioned nucleic acid molecule, the lower limit of the total of the number of nucleotides excluding the aforementioned linker region (Lx) is, for example, 36, preferably 38. The upper limit thereof is, for example, 100, preferably 80, more preferably 60, further preferably 50. Specific examples of the full length of the number of nucleotides is, for example, 36 - 100, preferably 38 - 80, more preferably 42 - 60, further preferably 42 - 50.

In a more preferable embodiment, the single-stranded nucleic acid molecule of the present invention has the aforementioned linker region (Lx) with a non-nucleotide structure.

The aforementioned non-nucleotide structure is not particularly limited and, for example, polyalkylene glycol, pyrrolidine skeleton, piperidine skeleton and the like can be mentioned. As the aforementioned polyalkylene glycol, for example, polyethylene glycol can be mentioned.

As the aforementioned pyrrolidine skeleton, for example, the skeleton of a pyrrolidine derivative, wherein one or more carbons constituting the 5-membered ring of pyrrolidine are substituted, can be mentioned. When the carbon is substituted, it is preferably, for example, a carbon atom other than C-2 carbon. The aforementioned carbon may be, for example, substituted by nitrogen, oxygen or sulfur. The aforementioned pyrrolidine skeleton may also contain, for example, in the 5-membered ring of pyrrolidine, for example, a carbon-carbon double bond or a carbon-nitrogen double bond. In the aforementioned pyrrolidine skeleton, the carbon and nitrogen constituting the 5-membered ring of pyrrolidine may be bonded to, for example, hydrogen or the below-mentioned substituent. The aforementioned linker region (Lx) can be bonded to the aforementioned region (X) and the aforementioned region (Xc), for example, via any group of the

aforementioned pyrrolidine skeleton. It is any one carbon atom or nitrogen of the aforementioned 5-membered ring, preferably, the 2-position carbon (C-2) or nitrogen of the aforementioned 5-membered ring. Examples of the aforementioned pyrrolidine skeleton include proline skeleton, prolinol skeleton and the like. The aforementioned proline skeleton and prolinol skeleton and the like are also superior in the safety since they are, for example, substances present in living organisms and reductants thereof.

As the aforementioned piperidine skeleton, for example, the skeleton of a piperidine derivative, wherein one or more carbons constituting the 6-membered ring of piperidine are substituted, can be mentioned. When the carbon is substituted, it is preferably, for example, a carbon atom other than C-2 carbon. The aforementioned carbon may be, for example, substituted by nitrogen, oxygen or sulfur. The aforementioned piperidine skeleton may also contain, for example, in the 6-membered ring of piperidine, for example, a carbon-carbon double bond or a carbon-nitrogen double bond. In the aforementioned piperidine skeleton, the carbon and nitrogen constituting the 6-membered ring of piperidine may be bonded to, for example, a hydrogen group or the below-mentioned substituent. The aforementioned linker region (Lx) can be bonded to the aforementioned region (X) and the aforementioned region (Xc), for example, via any group of the aforementioned piperidine skeleton. It is any one carbon atom or nitrogen of the aforementioned 6-membered ring, more preferably, the 2-position carbon (C-2) or nitrogen of the aforementioned 6-membered ring.

The aforementioned linker region may be composed of, for example, the non-nucleotide residue having the aforementioned non-nucleotide structure only, or may contain the non-nucleotide residue having the aforementioned non-nucleotide structure and the nucleotide residue.

The aforementioned linker region is represented, for example, by the following formula (I):

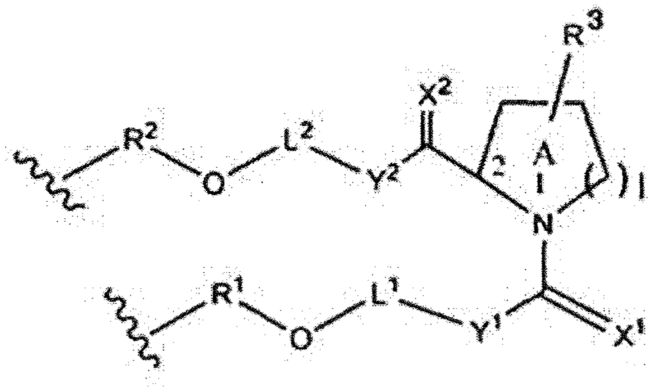

· · · (I)

In the aforementioned formula (I), for example,

$X^1$ and $X^2$ are each independently $H_2$, O, S, or NH;

$Y^1$ and $Y^2$ are each independently a single bond, $CH_2$, NH, O, or S;

$R^3$ is a hydrogen atom or a substituent bonded to C-3, C-4, C-5 or C-6 on ring A,

$L^1$ is an alkylene chain composed of n atoms, and a hydrogen atom on an alkylene carbon atom may or may not be substituted with OH, $OR^a$, $NH_2$, $NHR^a$, $NR^aR^b$, SH, or $SR^a$, or

$L^1$ is a polyether chain obtained by substituting at least one carbon atom on the aforementioned alkylene chain with an oxygen atom,

provided that: when $Y^1$ is NH, O, or S, an atom bound to $Y^1$ in $L^1$ is carbon, an atom bound to $OR^1$ in $L^1$ is carbon, and oxygen atoms are not adjacent to each other;

$L^2$ is an alkylene chain composed of m atoms, and a hydrogen atom on an alkylene carbon atom may or may not be substituted with OH, $OR^c$, $NH_2$, $NHR^c$, $NR^cR^d$, SH, or $SR^c$, or

$L^2$ is a polyether chain obtained by substituting at least one carbon atom on the aforementioned alkylene chain with an oxygen atom,

provided that: when $Y^2$ is NH, O, or S, an atom bound to $Y^2$ in $L^2$ is carbon, an atom bound to $OR^2$ in $L^2$ is carbon, and oxygen atoms are not adjacent to each other;

$R^a$, $R^b$, $R^c$, and $R^d$ are each independently a substituent or a protecting group;

1 is 1 or 2;

m is an integer in the range from 0 to 30;

n is an integer in the range from 0 to 30;

in ring A, one carbon atom other than C-2 on the aforementioned ring A may be substituted by nitrogen, oxygen or sulfur, the aforementioned ring A may contain a carbon-carbon double bond or a carbon-nitrogen double bond,

the aforementioned regions (Xc) and (X) are each linked to the aforementioned linker region (Lx) via $-OR^1-$ or $-OR^2-$, wherein $R^1$ and $R^2$ may or may not be present, and when they are present, $R^1$ and $R^2$ are each independently a nucleotide residue or the aforementioned structure (I).

In the aforementioned formula (I), for example, $X^1$ and $X^2$ are each independently $H_2$, O, S, or NH. In the aforementioned formula (I), "$X^1$ is $H_2$" means that $X^1$ forms $CH_2$ (a methylene group) together with a carbon atom to which $X^1$ binds. The same applies to $X^2$.

In the aforementioned formula (I), $Y^1$ and $Y^2$ are each independently a single bond, $CH_2$, NH, O, or S.

In the aforementioned formula (I), in ring A, 1 is 1 or 2. When 1 = 1, ring A is a 5-membered ring, for example, the aforementioned pyrrolidine skeleton. The aforementioned pyrrolidine skeleton is, for example, proline skeleton, prolinol skeleton or the like, and exemplified by the divalent structures thereof. When 1 = 2, ring A is a 6-membered ring, for example, the aforementioned piperidine skeleton. In ring A, one carbon atom other than C-2 on ring A may be substituted by nitrogen, oxygen or sulfur. Ring A may contain, in ring A, a carbon-carbon double bond or a carbon-nitrogen double bond. Ring A is, for example, L type or D type.

In the aforementioned formula (I), $R^3$ is a hydrogen atom or a substituent bonded to C-3, C-4, C-5 or C-6 on ring A. When $R^3$ is the aforementioned substituent, substituent $R^3$ may be one or plural or absent and, when it is in plurality, they may be the same or different.

The substituent $R^3$ is, for example, halogen, OH, $OR^4$, $NH_2$, $NHR^4$, $NR^4R^5$, SH, $SR^4$ or an oxo group (=O) and the like.

For example, $R^4$ and $R^5$ are each independently a substituent or a protecting group, and may be the same or different. Examples of the aforementioned substituent include halogen, alkyl, alkenyl, alkynyl, haloalkyl, aryl, heteroaryl, arylalkyl, cycloalkyl, cycloalkenyl, cycloalkylalkyl, cyclyl alkyl, hydroxyalkyl, alkoxyalkyl, aminoalkyl, heterocyclylalkenyl, heterocyclylalkyl, heteroarylalkyl, silyl, silyloxyalkyl and the like. Hereinafter the same applies. The substituent $R^3$ may be among these recited substituents.

The aforementioned protecting group is, for example, a functional group that inactivates a highly-reactive functional group. Examples of the protecting group include known protecting groups. Regarding the aforementioned protecting group, for example, the description in the literature (J.F.W. McOmie, "Protecting Groups in Organic Chemistry", Prenum Press, London and New York, 1973) can be incorporated herein. The aforementioned protecting group is not particularly limited, and examples thereof include a tert-butyldimethylsilyl group (TBDMS), a bis(2-acetoxyethyloxy)methyl group (ACE), a triisopropylsilyloxymethyl group (TOM), a 1-(2-cyanoethoxy)ethyl group (CEE), a 2-cyanoethoxymethyl group (CEM), a tolylsulfonylethoxymethyl group (TEM), and a dimethoxytrityl group (DMTr). When $R^3$ is $OR^4$, the aforementioned protecting group is not particularly limited, and examples thereof include a TBDMS group, an ACE group, a TOM group, a CEE group, a CEM group, and a TEM group. The same applies hereinafter.

In the aforementioned formula (I), $L^1$ is an alkylene chain composed of n atoms. A hydrogen atom(s) on the aforementioned alkylene carbon atom(s) may or may not be substituted with, for example, OH, $OR^a$, $NH_2$, $NHR^a$, $NR^aR^b$, SH, or $SR^a$. Alternatively, $L^1$ may be a polyether chain obtained by substituting at least one carbon atom on the aforementioned alkylene chain with an oxygen atom. The aforementioned polyether chain is, for example, polyethylene glycol. When $Y^1$ is NH, O, or S, an atom bound to $Y^1$ in $L^1$ is carbon, an atom bound to $OR^1$ in $L^1$ is carbon, and oxygen atoms are not adjacent to each other. That is, for example, when $Y^1$ is O, this oxygen atom and the oxygen atom in $L^1$ are not adjacent to each other, and the oxygen atom in $OR^1$ and the oxygen atom in $L^1$ are not adjacent to each other.

In the aforementioned formula (I), $L^2$ is an alkylene chain composed of m atoms. A hydrogen atom(s) on the aforementioned alkylene carbon atom(s) may or may not be substituted with, for example, OH, $OR^c$, $NH_2$, $NHR^c$, $NR^cR^d$, SH, or $SR^c$. Alternatively, $L^2$ may be a polyether chain obtained by substituting at least one carbon atom on the aforementioned alkylene chain with an oxygen atom. When $Y^2$ is NH, O, or S, an atom bound to $Y^2$ in $L^2$ is carbon, an atom bound to $OR^2$ in $L^2$ is carbon, and oxygen atoms are not adjacent to each other. That is, for example, when $Y^2$ is O, this oxygen atom and the oxygen atom in $L^2$ are not adjacent to each other, and the oxygen atom in $OR^2$ and the oxygen atom in $L^2$ are not adjacent to each other.

n of $L^1$ and m of $L^2$ are not particularly limited, and the lower limit of each of them may be 0, for example, and the upper limit of the same is not particularly limited. For example, n and m can be set as appropriate depending on a desired length of the aforementioned linker region (Lx). For example, from the view point of manufacturing cost, yield, and the like, n and m are each preferably 0 to 30, more preferably 0 to 20, and still more preferably 0 to 15. n and m may be the same (n = m) or different. n + m is, for example, 0 to 30, preferably 0 to 20, and more preferably 0 to 15.

$R^a$, $R^b$, $R^c$ and $R^d$ are, for example, each independently a substituent or a protecting group. Examples of the aforementioned substituent and the aforementioned protecting group are the same as those mentioned above.

In the aforementioned formula (I), hydrogen atoms, for example, each independently may be substituted with a halogen such as Cl, Br, F, or I.

The aforementioned regions (Xc) and (X) are linked to the aforementioned linker region (Lx) via $-OR^1-$ or $-OR^2-$, respectively. $R^1$ and $R^2$ may or may not be present. When $R^1$ and $R^2$ are present, $R^1$ and $R^2$ are each independently a nucleotide residue or the structure represented by the aforementioned formula (I). When $R^1$ and/or $R^2$ are/is the afore-

mentioned nucleotide residue, the aforementioned linker region (Lx) is composed of, for example, the aforementioned non-nucleotide residue having the structure of the aforementioned formula (I) excluding the nucleotide residue $R^1$ and/or $R^2$, and the aforementioned nucleotide residue (s) . When $R^1$ and/or $R^2$ are/is the structure represented by the aforementioned formula (I), the structure of the aforementioned linker region (Lx) is such that, for example, two or more of the aforementioned non-nucleotide residues having the structure of the aforementioned formula (I) are linked to each other. The number of the structures of the aforementioned formula (I) may be, for example, 1, 2, 3, or 4. When the linker region (Lx) includes a plurality of the aforementioned structures, the structures of the aforementioned (I) are preferably linked directly. On the other hand, when $R^1$ and $R^2$ are not present, the aforementioned linker region (Lx) is composed of the aforementioned non-nucleotide residue having the structure of the aforementioned formula (I) alone.

The combination of the aforementioned regions (Xc) and (X) with aforementioned $-OR^1-$ and $-OR^2-$ are not particularly limited, and may be, for example, any of the following conditions:

Condition (1):

The aforementioned regions (Xc) and (X) are linked to the structure of the aforementioned formula (I) via $-OR^2-$ and $-OR^1-$, respectively.

Condition (2)

The aforementioned regions (Xc) and (X) are linked to the structure of the aforementioned formula (I) via $-OR^1-$ and $-OR^2-$, respectively.

Examples of the structure of the aforementioned formula (I) include the structures of the following formulae (I-1) to (I-9). In the following formulae, n and m are the same as in the aforementioned formula (I). In the following formulae, q is an integer of 0 - 10.

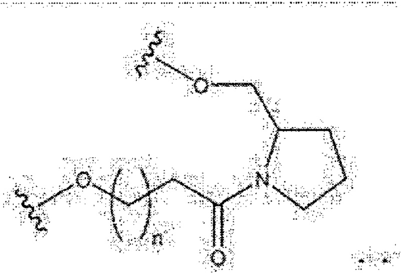

· · · (I-1)

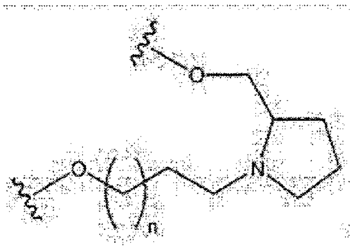

· · · (I-2)

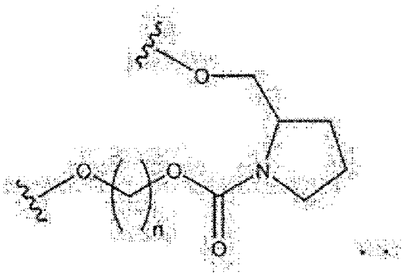

· · · (I-3)

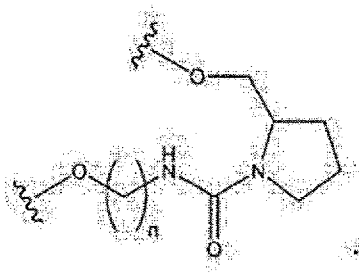

· · · (I-4)

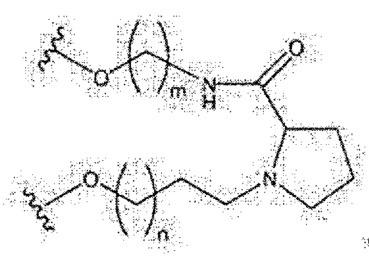

· · · (I-5)

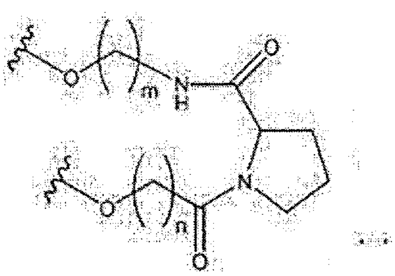

· · · (I-6)

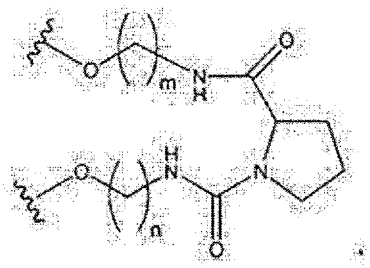

· · · (1-7)

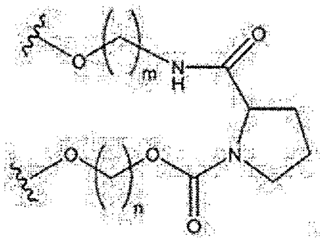

· · · (1-8)

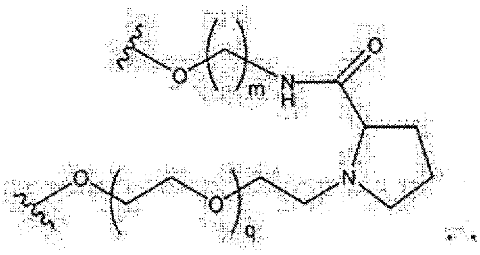

· · · (1-9)

In the aforementioned formulae (I-1) to (I-9), n, m and q are not particularly limited, and are as described above. Specific examples thereof include the aforementioned formula (I-1) wherein n=8, the aforementioned (1-2) wherein n=3, the aforementioned formula (1-3) wherein n=4 or 8, the aforementioned (1-4) wherein n=7 or 8, the aforementioned formula (I-5) wherein n=3 and m=4, the aforementioned (I-6) wherein n=8 and m=4, n=5 and m=4, the aforementioned formula (1-7) wherein n=8 and m=4, the aforementioned (1-8) wherein n=5 and m=4, n=8 and m=4, and the aforementioned formula (1-9) wherein q=1 and m=4. One embodiment (n=8) of the aforementioned formula (1-4) is shown in the following formula (I-4a), and one embodiment (n=5, m=4) of the aforementioned formula (I-6) is shown in the following formula (I-6a).

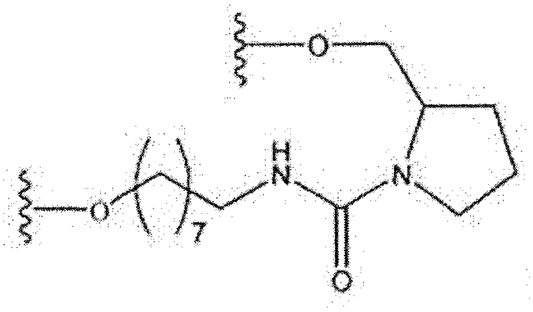

· · · (1-4a)

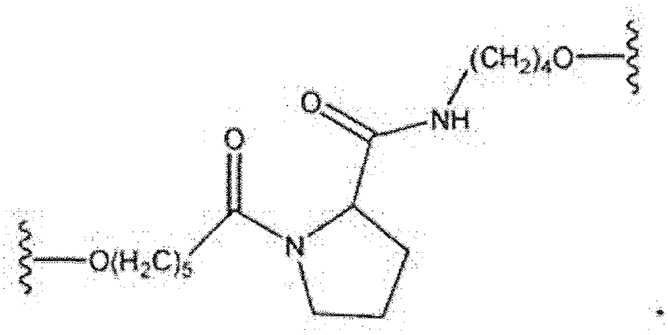

· · · (1-6a)

In a particularly preferable embodiment, the nucleic acid molecule of the present invention has any of the following structural formulas.

(SEQ ID NO: 23)

5'-CCUAUCUUCGACAACAUCAUCCC-Lx-GGGAUGAUGUUGUCGAAGAUAGGGG-3'
(SEQ ID NO: 24)
5'-GGAAUUUCCACUAUAUCAACCCC-Lx-GGGGUUGAUAUAGUGGAAAUUCCCU-3'
(SEQ ID NO: 25)
5'-GGGAAUUUCCACUAUAUCAACCC-Lx-GGGUUGAUAUAGUGGAAAUUCCCUU-3'
(SEQ ID NO: 26)
5'-CCUUCAUCCGAAAGUUCUACACC-Lx-GGUGUAGAACUUUCGGAUGAAGGUG-3'
(SEQ ID NO: 27)
5'-GUUCUACACAGAGUUUGAUCGCC-Lx-GGCGAUCAAACUCUGUGUAGAACUU-3'
(SEQ ID NO: 28)
5'-GUUGUUUUCCGAAAUGGAAAUCC-Lx-GGAUUUCCAUUUCGGAAAACAACAG-3'
(SEQ ID NO: 29)
5'-GUUUUCCGAAAUGGAAAUUGGCC-Lx-GGCCAAUUUCCAUUUCGGAAAACAA-3'
(SEQ ID NO: 30)
5'-CCUAUAACCUUGCAUAUAAGUCC-Lx-GGACUUAUAUGCAAGGUUAUAGGGA-3'
(SEQ ID NO: 31)
5'-CCCUAUAACCUUGCAUAUAAGCC-Lx-GGCUUAUAUGCAAGGUUAUAGGGAC-3'
(SEQ ID NO: 32)
5'-GGAGAAUGCAGUUCCUUUUAGCC-Lx-GGCUAAAAGGAACUGCAUUCUCCAA-3'
(SEQ ID NO: 33)
5'-GGGCUAAUAUGGAUACUAAAACC-Lx-GGUUUUAGUAUCCAUAUUAGCCCAU-3'
wherein Lx is any of the aforementioned linker regions.
Preferably, Lx has a structure of the above-mentioned formula (I), more preferably, any of the above-mentioned formulas (I-1) - (I-9), further preferably, the above-mentioned formula (I-4a) or (I-6a), particularly preferably, the above-mentioned formula (I-6a).

Among these, a single-stranded nucleic acid molecule composed of the nucleotide sequences shown in SEQ ID NO: 30 - 32 is particularly preferable.

The constitutional units of the the nucleic acid molecule of the present invention are not particularly limited and nucleotide residues can be mentioned. Examples of the aforementioned nucleotide residues include a ribonucleotide residue and a deoxyribonucleotide residue. The aforementioned nucleotide residue may be, for example, the one that is not modified (unmodified nucleotide residue) or the one that has been modified (modified nucleotide residue). By configuring the nucleic acid molecule of the present invention to include the aforementioned modified nucleotide residue, for example, the resistance of the nucleic acid molecule to nuclease can be improved, thereby allowing the stability of the nucleic acid molecule to be improved. Furthermore, the nucleic acid molecule of the present invention further may include, for example, a non-nucleotide residue in addition to the aforementioned nucleotide residue.

In the nucleic acid molecule of the present invention, the constitutional unit of each of the region other than aforementioned linker is preferably the aforementioned nucleotide residue. Each of the aforementioned regions is composed of, for example, any of the following residues (1) to (3) :

(1) an unmodified nucleotide residue(s)
(2) a modified nucleotide residue(s)
(3) an unmodified nucleotide residue(s) and a modified nucleotide residue(s).

In the nucleic acid molecule of the present invention, the constitutional unit of the aforementioned linker region is not particularly limited, and examples thereof include the aforementioned nucleotide residues and the aforementioned non-nucleotide residues. The aforementioned linker region may be composed of, for example, the aforementioned nucleotide residue only, the aforementioned non-nucleotide residue only, or both the aforementioned nucleotide residue and the aforementioned non-nucleotide residue. The aforementioned linker region is composed of, for example, any of the following residues (1) to (7) :

(1) an unmodified nucleotide residue(s)
(2) a modified nucleotide residue(s)
(3) an unmodified nucleotide residue(s) and a modified nucleotide residue(s)
(4) a non-nucleotide residue(s)
(5) a non-nucleotide residue(s) and an unmodified nucleotide residue(s)
(6) a non-nucleotide residue(s) and a modified nucleotide residue(s)
(7) a non-nucleotide residue(s), an unmodified nucleotide residue(s), and a modified nucleotide residue(s).

Examples of the nucleic acid molecule of the present invention include molecules composed of the aforementioned nucleotide residues only; and molecules including the aforementioned non-nucleotide residue(s) in addition to the aforementioned nucleotide residues. In the nucleic acid molecule of the present invention, for example, the aforementioned nucleotide residues may be the aforementioned unmodified nucleotide residues only; the aforementioned modified nucleotide residues only; or both the aforementioned unmodified nucleotide residue(s) and the aforementioned modified nucleotide residue(s), as described above. When the aforementioned nucleic acid molecule includes both the aforementioned unmodified nucleotide residue(s) and the aforementioned modified nucleotide residue(s), the number of the aforementioned modified nucleotide residue(s) is not particularly limited, and is, for example, "one to several", specifically, for example, 1 to 5, preferably 1 to 4, more preferably 1 to 3, and most preferably 1 or 2. When the nucleic acid molecule of the present invention include the aforementioned non-nucleotide residue(s), the number of the aforementioned non-nucleotide residue(s) is not particularly limited, and is, for example, "one to several", specifically, for example, 1-8, 1-6, 1-4, 1, 2 or 3.

When the aforementioned nucleic acid molecule includes, for example, the aforementioned modified ribonucleotide residue(s) in addition to the aforementioned unmodified ribonucleotide residues, the number of the aforementioned modified ribonucleotide residue(s) is not particularly limited, and is, for example, "one to several", specifically, for example, 1 to 5, preferably 1 to 4, more preferably 1 to 3, and most preferably 1 or 2. The aforementioned modified ribonucleotide residue as contrasted to the aforementioned unmodified ribonucleotide residue may be, for example, the aforementioned deoxyribonucleotide residue obtained by substituting a ribose residue with a deoxyribose residue. When the aforementioned nucleic acid molecule includes, for example, the aforementioned deoxyribonucleotide residue(s) in addition to the aforementioned unmodified ribonucleotide residue(s), the number of the aforementioned deoxyribonucleotide residue(s) is not particularly limited, and is, for example, "one to several", specifically, for example, 1 to 5, preferably 1 to 4, more preferably 1 to 3, and most preferably 1 or 2.

The nucleic acid molecule of the present invention may include, for example, a labeling substance, and may be labeled with the aforementioned labeling substance. The aforementioned labeling substance is not particularly limited, and may be, for example, a fluorescent substance, a dye, an isotope, or the like. Examples of the aforementioned labeling substance include: fluorophores such as pyrene, TAMRA, fluorescein, a Cy3 dye, and a Cy5 dye. Examples of the aforementioned dye include Alexa dyes such as Alexa 488. Examples of the aforementioned isotope include stable isotopes and radioisotopes. Among them, stable isotopes are preferable. For example, the aforementioned stable isotopes have a low risk of radiation exposure and they require no dedicated facilities. Thus, stable isotopes are excellent in handleability and can contribute to cost reduction. Moreover, for example, the aforementioned stable isotope does not change the physical properties of a compound labeled therewith and thus has an excellent property as a tracer. The aforementioned stable isotope is not particularly limited, and examples thereof include $^{2}H$, $^{13}C$, $^{15}N$, $^{17}O$, $^{18}O$, $^{33}S$, $^{34}S$, and, $^{36}S$.

2. Nucleotide residue constituting single-stranded nucleic acid molecule

The aforementioned nucleotide residue includes, for example, a sugar, a base, and a phosphate as its components. The aforementioned nucleotide residue may be, for example, a ribonucleotide residue or a deoxyribonucleotide residue, as described above. The aforementioned ribonucleotide residue has, for example, a ribose residue as the sugar; and adenine (A), guanine (G), cytosine (C), or uracil (U) as the base. The aforementioned deoxyribose residue has, for example, a deoxyribose residue as the sugar; and adenine (A), guanine (G), cytosine (C), or thymine (T) as the base.

The aforementioned nucleotide residue may be an unmodified nucleotide residue or a modified nucleotide residue. The aforementioned components of the aforementioned unmodified nucleotide residue are the same or substantially the same as, for example, the components of a naturally-occurring nucleotide residue. Preferably, the components are the same or substantially the same as the components of a nucleotide residue occurring naturally in a human body.

The aforementioned modified nucleotide residue is, for example, a nucleotide residue obtained by modifying the aforementioned unmodified nucleotide residue. For example, the aforementioned modified nucleotide residue may be such that any of the components of the aforementioned unmodified nucleotide residue is modified. In the present invention, "modification" means, for example, substitution, addition, and/or deletion of any of the aforementioned components; and substitution, addition, and/or deletion of an atom(s) and/or a functional group(s) in the aforementioned component(s). It can also be referred to as "modification". Examples of the aforementioned modified nucleotide residue include naturally-occurring nucleotide residues and artificially-modified nucleotide residues. Regarding the aforementioned naturally-derived modified nucleotide residues, for example, Limbach et al. (Limbach et al., 1994, Summary: the modified nucleosides of RNA, Nucleic Acids Res. 22: pp. 2183 to 2196) can be referred to. The aforementioned modified nucleotide residue may be, for example, a residue of an alternative of the aforementioned nucleotide.

Examples of the modification of the aforementioned nucleotide residue include modification of a ribose-phosphate backbone (hereinafter referred to as a "ribophosphate backbone").

In the aforementioned ribophosphate backbone, for example, a ribose residue may be modified. In the aforementioned ribose residue, for example, the 2'-position carbon can be modified. Specifically, for example, a hydroxyl group bound to the 2'-position carbon can be substituted with hydrogen or halogen such as fluoro. By substituting the hydroxyl group bound to the aforementioned 2'-position carbon with hydrogen, it is possible to substitute the ribose residue with deoxyribose. The aforementioned ribose residue can be substituted with its stereoisomer, for example, and may be substituted with, for example, an arabinose residue.

The aforementioned ribophosphate backbone may be substituted with, for example, a non-ribophosphate backbone having a non-ribose residue and/or a non-phosphate. The aforementioned non-ribophosphate backbone may be, for example, the aforementioned ribophosphate backbone modified to be uncharged. Examples of an alternative obtained by substituting the ribophosphate backbone with the aforementioned non-ribophosphate backbone in the aforementioned nucleotide include morpholino, cyclobutyl, and pyrrolidine. Other examples of the aforementioned alternative include artificial nucleic acid monomer residues. Specific examples thereof include PNA (Peptide Nucleic Acid), LNA (Locked Nucleic Acid), and ENA (2'-0,4'-C-Ethylenebridged Nucleic Acid). Among them, PNA is preferable.

In the aforementioned ribophosphate backbone, for example, a phosphate group can be modified. In the aforementioned ribophosphate backbone, a phosphate group at the closest adjacency to the sugar residue is called an "$\alpha$-phosphate group". The aforementioned $\alpha$-phosphate group is charged negatively, and the electric charges are distributed evenly over two oxygen atoms that are not linked to the sugar residue. Among the four oxygen atoms in the aforementioned $\alpha$-phosphate group, the two oxygen atoms not linked to the sugar residue in the phosphodiester linkage between the nucleotide residues hereinafter are referred to as "non-linking oxygens". On the other hand, two oxygen atoms that are linked to the sugar residue in the phosphodiester linkage between the aforementioned nucleotide residues hereinafter are referred to as "linking oxygens". For example, the aforementioned $\alpha$-phosphate group is preferably modified to be uncharged, or to render the charge distribution between the aforementioned non-linking oxygen asymmetric.

In the aforementioned phosphate group, for example, the aforementioned non-linking oxygen(s) may be substituted. The aforementioned oxygen(s) can be substituted with, for example, any atom selected from S (sulfur), Se (selenium), B (boron), C (carbon), H (hydrogen), N (nitrogen), and OR (R is an alkyl group or an aryl group) and substitution with S is preferable. It is preferable that both the aforementioned non-linking oxygens are substituted, for example, and it is more preferable that both the non-linking oxygens are substituted with S. Examples of the aforementioned modified phosphate group include phosphorothioates, phosphorodithioates, phosphoroselenates, boranophosphates, boranophosphates ester, hydrogen phosphonates, phosphoroamidates, alkyl or aryl phosphonates, and phosphotriesters. In particular, phosphorodithioate in which both of the aforementioned two non-linking oxygens are substituted with S is preferable.

In the aforementioned phosphate group, for example, the aforementioned linking oxygen(s) may be substituted. The aforementioned oxygen(s) can be substituted with, for example, any atom selected from S (sulfur), C (carbon), and N (nitrogen). Examples of the aforementioned modified phosphate group include: bridged phosphoroamidates resulting from the substitution with N; bridged phosphorothioates resulting from the substitution S; and bridged methylenephosphonates resulting from the substitution C. Preferably, substitution of the aforementioned linking oxygen(s) is performed in, for example, at least one of the 5'-terminus nucleotide residue and the 3'-terminus nucleotide residue of the nucleic acid molecule of the present invention. When the substitution is performed on the 5'-side, substitution with C is preferable. When the substitution is performed on the 3'-side, substitution with N is preferable.

The aforementioned phosphate group may be substituted with, for example, the aforementioned phosphate-free linker. The aforementioned linker may contain siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, methyleneoxymethylimino, or the like. Preferably, the linker may contain a methylenecarbonylamino group and a methylenemethylimino group.

In the nucleic acid molecule of the present invention, for example, at least one of a nucleotide residue at the 3'-terminus and a nucleotide residue at the 5'-terminus may be modified. For example, the nucleotide residue at either one of the 3'-terminus and the 5'-terminus may be modified, or the nucleotide residues at both the 3'-terminus and the 5'-terminus may be modified. The aforementioned modification may be, for example, as described above, and it is preferable to modify a phosphate group(s) at the end(s). For example, the entire aforementioned phosphate group may be modified, or one or more atoms in the aforementioned phosphate group may be modified. In the former case, for example, the entire phosphate group may be substituted or deleted.

Modification of the aforementioned nucleotide residue(s) at the end(s) may be, for example, addition of any other molecule. Examples of the aforementioned other molecule include functional molecules such as labeling substances as described above and protecting groups. Examples of the aforementioned protecting groups include S (sulfur), Si (silicon), B (boron), and ester-containing groups. The functional molecules such as the aforementioned labeling substances can be used, for example, in the detection and the like of the nucleic acid molecule of the present invention.

The aforementioned other molecule may be, for example, added to the phosphate group of the aforementioned nucleotide residue or may be added to the aforementioned phosphate group or the aforementioned sugar residue via

a spacer. For example, the terminus atom of the aforementioned spacer can be added to or substituted for either one of the aforementioned linking oxygens of the aforementioned phosphate group, or O, N, S, or C of the sugar residue. The binding site in the aforementioned sugar residue preferably is, for example, C at the 3'-position, C at the 5'-position, or any atom bound thereto. For example, the aforementioned spacer can also be added to or substituted for a terminus atom of the aforementioned nucleotide alternative such as PNA.

The aforementioned spacer is not particularly limited, and examples thereof include $-(CH_2)_n-$, $-(CH_2)_nN-$, $-(CH_2)_nO-$,$-(CH_2)_nS-$, $O(CH_2CH_2O)_nCH_2CH_2OH$, abasic sugars, amide, carboxy, amine, oxyamine, oxyimine, thioether, disulfide, thiourea, sulfonamide, and morpholino, and also biotin reagents and fluorescein reagents. In the aforementioned formulae, n is a positive integer, and n = 3 or 6 is preferable.

Other examples of the aforementioned molecule to be added to the end include dyes, intercalating agents (e.g., acridines), crosslinking agents (e.g., psoralen, mitomycin C), porphyrins (TPPC4, texaphyrin, sapphyrin), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrenebutyric acid, dihydrotestosterone, 1,3-Bis-O (hexadecyl)glycerol, a geranyloxyhexyl group, hexadecylglycerol, borneol, menthol, 1,3-propanediol, a heptadecyl group, palmitic acid, myristic acid, O3-(oleoyl)lithocholic acid, O3-(oleoyl)cholic acid, dimethoxytrityl, or phenoxathiine), peptide complexes (e.g., Antennapedia peptide, Tat peptide), alkylating agents, phosphate, amino, mercapto, PEG (e.g., PEG-40K), MPEG, $[MPEG]_2$, polyamino, alkyl, substituted alkyl, radiolabeled markers, enzymes, haptens (e.g., biotin), transport/absorption facilitators (e.g., aspirin, vitamin E, folic acid), and synthetic ribonucleases (e.g., imidazole, bisimidazole, histamine, imidazole clusters, acridine-imidazole complexes, $Eu^{3+}$ complexes of tetraazamacrocycles).

In the nucleic acid molecule of the present invention, the aforementioned 5'-terminus may be, for example, modified by a phosphoric acid group or a phosphoric acid group analog. Examples of the aforementioned phosphoric acid group include 5'-monophosphate ($(HO)_2(O)P-O-5'$); 5'-diphosphate ($(HO)_2(O)P-O-P(HO)(O)-O-5'$); 5'-triphosphate ($(HO)_2(O)P-O-(HO)(O)P-O-P(HO)(O)-O-5'$); 5'-guanosine cap (7-methylated or non-methylated, 7m-G-O-5'-(HO)(O)P-O-(HO)(O)P-O-P(HO)(O)-O-5'); 5'-adenosine cap (Appp); any modified or unmodified nucleotide cap structure (N-O-5'-(HO)(O)P-O-(HO)(O)P-O-P(HO)(O)-O-5'); 5'-monothiophosphate (phosphorothioate: $(HO)_2(S)P-O-5'$); 5'-dithiophosphate (phosphorodithioate: (HO)(HS)(S)P-O-5'); 5'-phosphorothiolate ($(HO)_2(O)P-S-5'$); sulfur substituted monophosphate, diphosphate, and triphosphates (e.g., 5'-$\alpha$-thiotriphosphate, 5'-$\gamma$-thiotriphosphate, and the like); 5'-phosphoramidates ($(HO)_2(O)P-NH-5'$, $(HO)(NH_2)(O)P-O-5'$); 5'-alkylphosphonates (e.g., RP(OH)(O)-O-5', $(OH)_2(O)P-5'-CH_2$, where R is alkyl (e.g., methyl, ethyl, isopropyl, propyl, or the like)); and 5'-alkyletherphosphonates (e.g., RP(OH)(O)-O-5', where R is alkylether (e.g., methoxymethyl, ethoxymethyl, or the like)).

In the aforementioned nucleotide residue, the aforementioned base is not particularly limited. The aforementioned base may be, for example, a natural base or a non-natural base. The aforementioned base may be, for example, a naturally-derived base or a synthetic base. As the aforementioned base, for example, a common base, a modified analog thereof, and the like can be used.

Examples of the aforementioned base include: purine bases such as adenine and guanine; and pyrimidine bases such as cytosine, uracil, and thymine. Other examples of the aforementioned base include inosine, thymine, xanthine, hypoxanthine, nubularine, isoguanisine, and tubercidine. Examples of the aforementioned base also include: 2-aminoadenine, alkyl derivatives such as 6-methylated purine; alkyl derivatives such as 2-propylated purine; 5-halouracil and 5-halocytosine; 5-propynyluracil and 5-propynylcytosine; 6-azouracil, 6-azocytosine, and 6-azothymine; 5-uracil (pseudouracil), 4-thiouracil, 5-halouracil, 5-(2-aminopropyl)uracil, 5-aminoallyluracil; 8-halogenated, aminated, thiolated, thioalkylated, hydroxylated, and other 8-substituted purines; 5-trifluoromethylated and other 5-substituted pyrimidines; 7-methylguanine; 5-substituted pyrimidines; 6-azapyrimidines; N-2, N-6, and O-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazoles; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N4-acetylcytosine; 2-thiocytosine; N6-methyladenine; N6-isopentyladenine; 2-methylthio-N6-isopentenyladenine; N-methylguanine; and O-alkylated bases. Examples of the purines and pyrimidines include those disclosed in U.S. Patent No. 3,687,808, "Concise Encyclopedia of Polymer Science and Engineering", pp. 858 to 859, edited by Kroschwitz J. I, John Wiley & Sons, 1990, and Englisch et al, Angewandte Chemie, International Edition, 1991, vol. 30, p. 613.

Other examples of the aforementioned modified nucleotide residue include those having no base, i.e., those having an abasic ribophosphate backbone. Furthermore, as the aforementioned modified nucleotide residue, for example, those described in U.S. Provisional Application No. 60/465,665 (filing date: April 25, 2003) and International Application No. PCT/US04/07070 (filing date: March 8, 2004) can be used and these documents are incorporated herein by reference.

3. Synthesis method of nucleic acid molecule of the present invention

The method for synthesizing the nucleic acid molecule of the present invention is not particularly limited, and a conventionally known method can be employed. Examples of the aforementioned synthesis method include synthesis methods according to genetic engineering procedures and chemical synthesis methods. Examples of the genetic engineering procedures include: synthesis methods utilizing in vitro transcription; methods using a vector; methods carried out using a PCR cassette and the like. The aforementioned vector is not particularly limited, and examples thereof include non-virus vectors such as plasmid, and virus vectors. The aforementioned chemical synthesis methods are not particularly limited, and examples thereof include a phosphoramidite method and an H-phosphonate method. The aforementioned chemical synthesis methods can be carried out, for example, using a commercially available automated nucleic acid synthesizer. In the aforementioned chemical synthesis methods, an amidite is generally used. The aforementioned amidite is not particularly limited. Examples of commercially available amidites include RNA Phosphoramidites (2'-O-TBDMSi, trade name, Samchully Pharm. Co., Ltd.), ACE amidite and TOM amidite, CEE amidite, CEM amidite, and TEM amidite and the like.

4. Expression vector

When the nucleic acid molecule of the present invention is constituted solely of a non-modified ribonucleotide residue, it can also be provided in the form of a vector encoding the nucleic acid molecule in an expressible state (expression vector of the present invention) as a precursor of the nucleic acid molecule. The expression vector of the present invention characteristically contains DNA encoding the aforementioned nucleic acid molecule of the present invention in the target cell under regulation of a functional promoter. The expression vector of the present invention is characterized in that it contains a promoter functionally linked to the aforementioned DNA, and other configurations are by no means limited. The vector to be inserted with the aforementioned DNA is not particularly limited and, for example, general vectors can be used such as virus vector, non-virus vector and the like. The aforementioned non-virus vector is, for example, plasmid vector. It is possible to inhibit expression of the prorenin gene or prorenin receptor gene in the cell by introducing the expression vector into the target cell (cell having the aforementioned prorenin gene and prorenin receptor gene) using a gene transfer method known per se.

5. Expression inhibitor

The expression inhibitor of the present invention is a preparation that inhibits expression of a prorenin gene or a prorenin receptor gene, and characterized in that it contains the aforementioned nucleic acid molecule of the present invention as an active ingredient.

The expression inhibitor of the present invention comprises a step of administering the aforementioned nucleic acid molecule singly or together with a pharmacologically acceptable carrier to, for example, a target in which the aforementioned prorenin gene and prorenin receptor gene are present. The aforementioned administration step is performed by, for example, contacting the aforementioned nucleic acid molecule with the aforementioned subject of administration. Examples of the aforementioned administration subject also include cells, tissues and organs of humans, nonhuman animals such as nonhuman mammals, i.e., mammals excluding humans. The aforementioned administration may be performed, for example, in vivo or in vitro.

6. Therapeutic agent for eye diseases

Prorenin-prorenin receptor are deeply involved in inflammation and angiogenesis in the ocular tissue through two different actions of tissue RAS activation and RAS-independent intracellular signaling activation (collectively referred to as receptor-associated prorenin system (RAPS)). Therefore, a medicament containing the nucleic acid molecule of the present invention as an active ingredient is effective for the treatment of eye diseases involving RAPS (e.g., diabetic retinopathy, uveitis, age-related macular degeneration etc.) since it inhibits RAPS. In addition, a medicament containing the nucleic acid molecule of the present invention as an active ingredient is effective for the treatment of diabetic retinopathy, uveitis, age-related macular degeneration or glaucoma. The "treatment" here is used in a meaning encompassing prophylaxis and delay of onset of disease, improvement of disease, and improvement of prognosis.

As the medicament of the present invention, an effective amount of the nucleic acid molecule of the present invention may be used alone, or can also be formulated as a pharmaceutical composition together with any carrier, for example, a pharmaceutically acceptable carrier.

Examples of the pharmaceutically acceptable carrier include, but are not limited to, excipients such as sucrose, starch and the like, binders such as cellulose, methylcellulose and the like, disintegrants such as starch, carboxymethylcellulose and the like, lubricants such as magnesium stearate, aerogel and the like, flavors such as citric acid, menthol

and the like, preservatives such as sodium benzoate, sodium bisulfite and the like, stabilizers such as citric acid, sodium citrate and the like, suspending agents such as methylcellulose, polyvinyl pyrrolidone and the like, dispersing agents such as surfactant and the like, diluents such as water, physiological saline and the like, base wax and the like.

To facilitate the introduction of the nucleic acid molecule of the present invention into the target cell, the pharmaceutical of the present invention can further comprise a reagent for nucleic acid introduction. Cationic lipids such as atelocollagen; liposome; nanoparticle; Lipofectin, Lipofectamine, DOGS (Transfectam), DOPE, DOTAP, DDAB, DHDEAB, HDEAB, polybrene, or poly(ethyleneimine) (PEI) and the like, and the like can be used as the reagent for nucleic acid introduction.

In one preferable embodiment, the pharmaceutical of the present invention may be a pharmaceutical composition wherein the nucleic acid molecule of the present invention is encapsulated in a liposome. A liposome is a microscopic closed vesicle having an internal phase enclosed by one or more lipid bilayers, and typically can retain a water-soluble substance in the internal phase and a lipophilic substance in the lipid bilayer. Herein, when the term "encapsulated" is used, the nucleic acid molecule of the present invention may be retained in the internal phase of the liposome or in the lipid bilayer. The liposome to be used in the present invention may be a single-layer film or a multi-layer film. The particle size of the liposome can be appropriately selected within the range of, for example, 10 - 1000 nm, preferably 50 - 300 nm. Considering the delivery efficiency to the target tissue, the particle size can be, for example, 200 nm or less, preferably 100 nm or less.

Methods of encapsulating a water-soluble compound such as nucleic acid into a liposome include lipid film method (vortex method), reversed-phase evaporation method, surfactant removal method, freeze-thawing method, remote loading method and the like, but are not limited thereto, and any known method can be appropriately selected.

The pharmaceutical of the present invention can be locally administered into eyes of a mammal. In particular, it is desirable to be instilled into eyes.

Preparations suitable for ocular topical administration include an eye drop (aqueous eye drop, non-aqueous eye drop, eye drop suspension, eye drop emulsion etc.), ointment, lotion, cream and the like. When the agent of the present invention is an eye drop, a base can be appropriately used. The bases to be used for eye drop include phosphate buffer, Hank's buffer, physiological saline, perfusion fluid, artificial lacrimal fluid and the like.

When the pharmaceutical of the present invention is a preparation for ocular topical administration, for example, buffering agent, isotonicity agent, solubilizing agent, preservative, viscosity base, chelating agent, algefacient, pH adjuster, antioxidant and the like can be selected and added as appropriate.

Examples of the buffering agent include phosphate buffering agent, borate buffering agent, citrate buffering agent, tartrate buffering agent, acetate buffering agent, amino acid and the like.

Examples of the isotonicity agent include saccharides such as sorbitol, glucose, mannitol and the like, polyvalent alcohols such as glycerol, propylene glycol and the like, salts such as sodium chloride and the like, boric acid and the like.

Examples of the solubilizing agent include non-ionic surfactants such as sorbitan polyoxyethylene monooleate (e.g., polysorbate80), polyoxyethylene hydrogenated castor oil, Tyloxapol, pluronic and the like, polyvalent alcohols such as glycerol, macrogol and the like, and the like.

Examples of the preservative include quaternary ammonium salts such as benzalkonium chloride, benzethonium chloride, cetyl pyridinium chloride and the like, paraoxybenzoates such as methyl p-hydroxybenzoate, ethyl parahydroxybenzoate, propyl p-hydroxybenzoate, butyl p-hydroxybenzoate and the like, benzyl alcohol, sorbic acid and a salt thereof (sodium salt, potassium salt and the like), thimerosal (trade name), chlorobutanol, sodium dehydroacetate and the like.

Examples of the viscosity base include water-soluble polymers such as polyvinylpyrrolidone, polyethylene glycol, poly(vinyl alcohol) and the like, celluloses such as hydroxyethylcellulose, methylcellulose, hydroxypropylmethylcellulose, sodium carboxymethylcellulose and the like, and the like.

Examples of the chelating agent include sodium edetate, citric acid and the like.

Examples of the algefacient include 1-menthol, borneol, camphor, eucalyptus oil and the like.

Examples of the pH adjuster include sodium hydroxide, potassium hydroxide, sodium carbonate, sodium hydrogen carbonate, boric acid or a salt thereof (borax), hydrochloric acid, citric acid or a salt thereof (sodium citrate, sodium dihydrogen citrate etc.), phosphoric acid or a salt thereof (disodium hydrogen phosphate, potassium dihydrogen phosphate etc.), acetic acid or a salt thereof (sodium acetate, ammonium acetate etc.), tartaric acid or a salt thereof (sodium tartrate etc.) and the like.

Examples of the antioxidant include sodium bisulfite, dried sodium sulfite, sodium pyrrosulfite, mixed tocopherols concentrate and the like.

The content of the nucleic acid molecule of the present invention in the pharmaceutical composition of the present invention is, for example, about 0.1 - 100 wt% of the total pharmaceutical composition.

When the pharmaceutical composition of the present invention is a liposome preparation, the molar ratio of the nucleic acid molecule of the present invention to liposome constituents is generally 1/100,000 - 1/10,000. The content of the liposome encapsulating the nucleic acid molecule of the present invention contained in the liposome preparation

is not particularly limited, as long as it is an amount in which liposome particles do not aggregate and sufficient efficacy can be exerted, and generally 10 - 100 mM.

The dose of the pharmaceutical of the present invention varies depending on the object of administration, method of administration, kind of ocular surface disorder, size of lesion, situation of the subject of administration (sex, age, body weight and the like). When the pharmaceutical of the present invention is instilled into eyes of an adult human, it is preferable to administer generally 0.01 - 1000 μg, preferably 0.05 - 100 μg, more preferably 0.1 - 50 μg, as a single dose of the nucleic acid of the present invention once to 10 times, preferably 5 - 10 times, per day.

In the following, the present invention will be described in detail with reference to examples and the like. It is to be noted, however, the present invention is by no means limited thereto.

[Examples]

(Example 1) Prorenin gene or prorenin receptor gene expression inhibitory effect by single-stranded nucleic acid molecule in HCC1954 cell

(1) Synthesis of single-stranded nucleic acid molecule

Single-stranded nucleic acid molecules shown below were synthesized based on the phosphoramidite method by ABI3900 nucleic acid synthesizer (trade name, Applied Biosystems). In the aforementioned synthesis, EMM amidite (WO 2013/027843) was used as RNA amidite (hereinafter the same). The aforementioned amidite was deprotected by a conventional method. The synthesized single-stranded nucleic acid molecules were purified by HPLC and respectively freeze-dried.

As single-stranded nucleic acid molecules, single-stranded nucleic acid molecules having a prorenin gene expression inhibitory sequence shown in the aforementioned SEQ ID NO: 1 to 5 (PH-0001-h-p, PH-0002-h-p, PH-0003-h-p, PH-0004-h-p, PH-0005-h-p), and single-stranded nucleic acid molecules having a prorenin receptor gene expression inhibitory sequence shown in the aforementioned SEQ ID NO: 6 to 11 (PH-0001-hmr-pr, PH-0002-hmr-pr, PH-0001-hm-pr, PH-0002-hm-pr, PH-0003-hm-pr, PH-0001-h-pr) are each synthesized as mentioned above. In each single-stranded nucleic acid molecule, Lx is a linker region Lx, and the following structural formulae were obtained using L-proline diamide amidite. In each sequence, the underlined is the human prorenin gene or prorenin receptor gene expression inhibitory sequence.

PH-0001-h-p (SEQ ID NO: 23)
5'-CCUAUCUUCGACAACAUCAUCCC-Lx-GGGAUGAUGUUGUCGAAGAUAGGGG-3'
PH-0002-h-p (SEQ ID NO: 24)
5'-GGAAUUUCCACUAUAUCAACCCC-Lx-GGGGUUGAUAUAGUGGAAAUUCCCU-3'
PH-0003-h-p (SEQ ID NO: 25)
5'-GGGAAUUUCCACUAUAUCAACCC-Lx-GGGUUGAUAUAGUGGAAAUUCCCUU-3'
PH-0004-h-p (SEQ ID NO: 26)
5'-CCUUCAUCCGAAAGUUCUACACC-Lx-GGUGUAGAACUUUCGGAUGAAGGUG-3'
PH-0005-h-p (SEQ ID NO: 27)
5'-GUUCUACACAGAGUUUGAUCGCC-Lx-GGCGAUCAAACUCUGUGUAGAACUU-3'
PH-0001-hmr-pr (SEQ ID NO: 28)
5'-GUUGUUUUCCGAAAUGGAAAUCC-Lx-GGAUUUCCAUUUCGGAAAACAACAG-3'
PH-0002-hmr-pr (SEQ ID NO: 29)
5'-GUUUUCCGAAAUGGAAAUUGGCC-Lx-GGCCAAUUUCCAUUUCGGAAAACAA-3'
PH-0001-hm-pr (SEQ ID NO: 30)
5'-CCUAUAACCUUGCAUAUAAGUCC-Lx-GGACUUAUAUGCAAGGUUAUAGGGA-3'
PH-0002-hm-pr (SEQ ID NO: 31)
5'-CCCUAUAACCUUGCAUAUAAGCC-Lx-GGCUUAUAUGCAAGGUUAUAGGGAC-3'
PH-0003-hm-pr (SEQ ID NO: 32)
5'-GGAGAAUGCAGUUCCUUUUAGCC-Lx-GGCUAAAAGGAACUGCAUUCUCCAA-3'
PH-0001-h-pr (SEQ ID NO: 33)
5'-GGGCUAAUAUGGAUACUAAAACC-Lx-GGUUUUAGUAUCCAUAUUAGCCCAU-3'

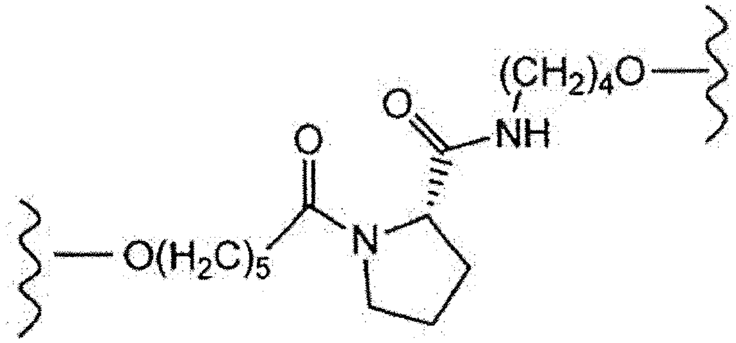

(2) Measurement of expression level of prorenin gene and prorenin receptor gene

The aforementioned each single-stranded nucleic acid molecule was dissolved in distilled water for injection (Otsuka Pharmaceutical Co., Ltd.) to 20 μmol/L.

As the cell, HCC1954 cell (ATCC) was used. As a medium, RPMI-1640 containing 10% FBS (Invitrogen) was used. The culture conditions were 37°C, 5% $CO_2$.

The cells were first seeded in a 24-well plate at $1\times10^4$ cells/400 μL medium/well and cultured for 24 hr. Thereafter, the cells were transfected with the aforementioned single-stranded nucleic acid molecule by using transfection reagent Lipofectamine RNAiMAX (Invitrogen) according to the protocol attached to the aforementioned transfection reagent. Specifically, the transfection was carried out by setting the composition per well as follows. In the following compositions, (A) is the aforementioned transfection reagent, (B) is Opti-MEM (Invitrogen), (C) is 20 μmol/L single-stranded nucleic acid molecule solution mentioned above. The final concentration of the aforementioned single-stranded nucleic acid molecule in the aforementioned well was set to 10 nmol/L.

Table 1

(Composition per well: μL)

| | |
|---|---|
| cultured broth | 400 |
| (A) + (B) | 50 |
| (B) + (C) | 50 |
| | 500 |

After the transfection, the cells in the aforementioned wells were cultured for 24 hr, and then, the RNA was collected using ISOGENII (NIPPON GENE) according to the protocol supplied therewith. Subsequently, cDNA was synthesized from the aforementioned RNA using Transcription First Strand cDNA Synthesis Kit (Roche) according to the protocol supplied therewith. Then, as shown below, PCR was carried out using the aforementioned synthesized cDNA as a template, and the expression level of the prorenin gene, the expression level of the prorenin receptor gene and the expression level of the internal standard, GAPDH gene, were measured. The expression level of the aforementioned prorenin gene and the expression level of the prorenin receptor gene were normalized with reference to that of the aforementioned GAPDH gene.

The aforementioned PCR was carried out using LightCycler 480 SYBR Green I Master (Roche) as a reagent and LightCycler 480 (Roche) as an instrument (hereinafter the same). The aforementioned prorenin gene, prorenin receptor gene and GAPDH gene were amplified using the following primer sets, respectively.

PCR primer set for prorenin gene

(SEQ ID NO: 34) 5'-GCTTTCTCAGCCAGGACATC-3'
(SEQ ID NO: 35) 5'-TGGGAGATGATGTTGTCGAA-3'

PCR primer set for prorenin receptor gene

(SEQ ID NO: 36) 5'-TCGTACCCTTTGGAGAATGC-3'
(SEQ ID NO: 37) 5'-GAGCCAACTGCAAAACAACA-3'

primer set for GAPDH gene

(SEQ ID NO: 38) 5'-GGAGAAGGCTGGGGCTCATTTGC-3'
(SEQ ID NO: 39) 5'-TGGCCAGGGGTGCTAAGCAGTTG-3'

As a control, regarding the cells subjected to the same transfection procedures as in the above except that the aforementioned single-stranded nucleic acid molecule solution (C) was not added and that the aforementioned (A) and the aforementioned (B) were added so that the total amount of (A) and (B) would be 100 μL, the expression level of the gene also was measured (mock).

As for the expression level of the prorenin gene and the expression level of the prorenin receptor gene after normalization, the relative value in the cell introduced with each single-stranded nucleic acid molecule was determined based on the expression level in the cells of the control (mock) set as 1.

(3) Results

The measurement results of the prorenin gene expression level are shown in Fig. 2, and the measurement results of the prorenin receptor gene expression level are shown in Fig. 3. The single-stranded nucleic acid molecule of the present invention showed a strong gene expression inhibitory activity.

(Example 2) Inhibitory effect on expression of prorenin receptor gene

The single-stranded nucleic acid molecules shown in SEQ ID NO: 28 and 29 and having an expression inhibitory sequence of human, mouse or rat prorenin receptor gene, and the single-stranded nucleic acid molecules shown in SEQ ID NO: 30 - 32 and having an expression inhibitory sequence of a human or mouse prorenin receptor gene were confirmed for an inhibitory effect on the expression of the prorenin receptor gene in cultured cells derived from human, mouse and rat.

(1) Measurement of expression level of prorenin receptor gene

As the cells, human-derived hTERT-RPE cell (ATCC), mouse-derived Neuro-2a cell (ECACC), rat Rat-1 cell (ATCC) were used. As each medium, DMEM/F12, E-MEM, DMEM medium (Wako Pure Chemical Industries, Ltd.) containing 10% FBS were used. The culture conditions were 37°C, 5% $CO_2$.

The cells were first seeded in a 96-well plate at $1 \times 10^4$ cells/80 μL medium/well and cultured for 24 hr. Thereafter, the cells were transfected with the aforementioned single-stranded nucleic acid molecule by using transfection reagent Lipofectamine RNAiMAX (Invitrogen) according to the protocol attached to the aforementioned transfection reagent. Specifically, the transfection was carried out by setting the composition per well as follows. In the following compositions, (A) is the aforementioned transfection reagent, (B) is Opti-MEM (Invitrogen), (C) is 20 μmol/L single-stranded nucleic acid molecule solution mentioned above. The final concentration of the aforementioned single-stranded nucleic acid molecule in the aforementioned well was set to 0.5 nmol/L.

Table 2

| (Composition per well: μL) | |
|---|---|
| cultured broth | 80 |
| (A) + (B) | 10 |
| (B) + (C) | 10 |
| | 100 |

After the transfection, the cells in the aforementioned wells were cultured for 24 hr, cDNA was synthesized from RNA by using SuperPrep Cell Lysis & RT (TOYOBO) according to the attached protocol. Then, as shown below, PCR was carried out using the aforementioned synthesized cDNA as a template, and the expression levels of the prorenin receptor gene and the internal standard HPRT1 gene were measured. The expression level of the aforementioned prorenin receptor gene was normalized with reference to that of the HPRT1 gene mentioned above.

In the aforementioned PCR, GoTaq qPCR Master Mix (Promega) was used as the reagent, and StepOne Plus (Life Techonologies) was used as the instrument. The prorenin receptor gene and the HPRT1 gene were amplified using the following primer sets.

PCR primer set for human prorenin receptor gene

(SEQ ID NO: 40) 5'-AGGCAGTGTCATTTCGTACC-3'
(SEQ ID NO: 41) 5'-GCCTTCCCTACCATATACACTC-3'

PCR primer set for human HPRT1 gene

(SEQ ID NO: 42) 5'-ACCCCACGAAGTGTTGGATA-3'
(SEQ ID NO: 43) 5'-AAGCAGATGGCCACAGAACT-3'

PCR primer set for mouse prorenin receptor gene

(SEQ ID NO: 44) 5'-TGGTCGTTCTCCTGTTCTTTC-3'
(SEQ ID NO: 45) 5'-ACCCTGGCGATCTTAATATGC-3'

PCR primer set for mouse HPRT1 gene

(SEQ ID NO: 46) 5'-CAAACTTTGCTTTCCCTGGT-3'
(SEQ ID NO: 47) 5'-CAAGGGCATATCCAACAACA-3'

PCR primer set for rat prorenin receptor gene

(SEQ ID NO: 48) 5'-TGTGGGCAACCTATTCCACC-3'
(SEQ ID NO: 49) 5'-AGCTGACGCAATGTGACTGA-3'

PCR primer set for Rat HPRT1 gene

(SEQ ID NO: 50) 5'-GCAGACTTTGCTTTCCTTGG-3'
(SEQ ID NO: 51) 5'-TCCACTTTCGCTGATGACAC-3'

As control, regarding the cells subjected to the same transfection procedures as in the above except that the aforementioned RNA solution (C) was not added and that the aforementioned (A) and (B) were added so that the total amount of (A) and (B) would be 20 μL, the expression level of the gene also was measured (mock).
As for the expression levels of normalized prorenin gene, the relative value of the expression level in the cell introduced with each single-stranded nucleic acid molecule was determined based on the expression level in the cells of the control (mock) as 1.

(2) Results

The measurement results of the expression level of human prorenin receptor gene are shown in Fig. 4, the results of the expression level of the mouse prorenin receptor gene are shown in Fig. 5, and the measurement results of the expression level of the rat prorenin receptor gene are shown in Fig. 6. In each cell, the single-stranded nucleic acid molecule of the present invention showed a strong gene expression inhibitory effect.

(Example 3) Inhibitory effect on expression of prorenin gene

The single-stranded nucleic acid molecules shown in SEQ ID NO: 23 to 27 and having an expression inhibitory sequence of human prorenin gene were confirmed for the expression inhibitory effect on the prorenin gene in human-derived cultured cells.

(1) Measurement of expression level of prorenin gene

As the cell, human-derived hTERT-RPE cell (ATCC) was used. As the medium, DMEM/F12 containing 10% FBS was used. The culture conditions were 37°C, 5% $CO_2$.
The cells were first seeded in a 24-well plate at $2\times10^4$ cells/400 μL medium/well and cultured for 24 hr. Thereafter, the cells were transfected with the aforementioned single-stranded nucleic acid molecule by using transfection reagent Lipofectamine RNAiMAX (Invitrogen) according to the protocol attached to the aforementioned transfection reagent. Specifically, the transfection was carried out by setting the composition per well as follows. In the following compositions, (A) is the aforementioned transfection reagent, (B) is Opti-MEM (Invitrogen), (C) is 20 μmol/L single-stranded nucleic acid molecule solution mentioned above. The final concentration of the aforementioned single-stranded

nucleic acid molecule in the aforementioned well was set to 0.5 nmol/L.

Table 3

| (Composition per well: μL) | |
|---|---|
| cultured broth | 400 |
| (A) + (B) | 50 |
| (B) + (C) | 50 |
| | 500 |

After the transfection, the cells in the aforementioned wells were cultured for 24 hr, and the RNA was collected using ISOGENII (NIPPON GENE) according to the attached protocol. Then, cDNA was synthesized from the aforementioned RNA using GoScript Reverse Transcriptase Kit (Promega) according to the attached protocol. Then, as shown below, PCR was carried out using the aforementioned synthesized cDNA as a template, and the expression level of the prorenin gene and that of the and HPRT1 gene as an internal standard were measured. The aforementioned expression level of the prorenin gene was normalized with reference to that of the aforementioned HPRT1 gene.

In the aforementioned PCR, GoTaq qPCR Master Mix (Promega) and THUNDERBIRD Probe qPCR Mix (TOYOBO) were used as the reagents, and StepOne Plus (Life Techonologies) was used as the instrument. For the measurement of the expression level of the aforementioned prorenin gene, Taqman Assay (Life Techonologies) was used and HPRT1 gene was amplified using the following primer set.
PCR primer set for human HPRT1 gene

(SEQ ID NO: 42) 5'-ACCCCACGAAGTGTTGGATA-3'
(SEQ ID NO: 43) 5'-AAGCAGATGGCCACAGAACT-3'

As a control, regarding the cells subjected to the same transfection procedures as in the above except that the aforementioned single-stranded nucleic acid molecule solution (C) was not added and that the aforementioned (A) and the aforementioned (B) were added so that the total amount of (A) and (B) would be 100 μL, the expression level of the gene also was measured (mock).

As for the expression level of the prorenin gene after normalization, the relative value in the cell introduced with each single-stranded nucleic acid molecule was determined based on the expression level in the cells of the control (mock) set as 1.

(2) Results

The results are shown in Fig. 7. The single-stranded nucleic acid molecule of the present invention showed a strong gene expression inhibitory effect.

(Example 4) Inflammation inhibitory effect in model mouse that developed endotoxin-induced uveitis

PH-0001-hm-pr, a single-stranded nucleic acid molecule shown in SEQ ID NO: 30 having an inhibitory sequence of expression of human and mouse prorenin receptor genes, was confirmed for an inflammation inhibitory effect in model mouse that developed LPS-induced uveitis.

(1) Administration of single-stranded nucleic acid molecule to endotoxin-induced uveitis model mouse

The aforementioned single-stranded nucleic acid molecule was dissolved in PBS to 30, 100, 300 μM. After dissolution, a single-stranded nucleic acid molecule solution (1 μL) at each concentration was intravitreously administered using a 33 gauge injection needle to the mice under pentobarbital anesthesia. As negative control, the same amount of PBS was used. In each administration group, 6 male mice (C57BL/6J, 6- to 8-week-old) were used. At 24 hr after administration of the single-stranded nucleic acid molecule, LPS (0.2 mg) dissolved in 100 μL of PBS was intraperitoneally administered.

(2) Measurement of expression level of inflammatory cytokine gene

At 6 hr after LPS administration, the mice were euthanized by cervical dislocation, retina was isolated, and RNA was collected using TRIzol (Life technologies) according to the attached protocol. Then, cDNA was synthesized

from the aforementioned RNA by using GoScript Reverse Transcriptase Kit (Promega) according to the attached protocol. Then, as shown below, PCR was carried out using the aforementioned synthesized cDNA as a template, and the expression levels of CCL2/MCP-1, ICAM-1, IL-6, TNF-$\alpha$ genes and the expression level of Gapdh gene as an internal standard was measured. The aforementioned expression level of each gene were normalized with reference to that of the aforementioned Gapdh gene.

In the aforementioned PCR, GoTaq qPCR Master Mix (Promega) was used as the reagent and StepOne Plus (Life Techonologies) was used as the instrument. In PCR of each gene, the following primer sets were used.

PCR primer set for mouse CCL2/MCP-1 gene

(SEQ ID NO: 52) 5'-TTGGCTCAGCCAGATGCA-3'
(SEQ ID NO: 53) 5'-CCTACTCATTGGGATCATCTTGC-3'

PCR primer set for mouse ICAM-1 gene

(SEQ ID NO: 54) 5'-CCTGTTTCCTGGCTCTGAAG-3'
(SEQ ID NO: 55) 5'-GTCTGCTGAGACCCCTCTTG-3'

PCR primer set for mouse IL-6 gene

(SEQ ID NO: 56) 5'-CACAGAGGATACCACTCCCAACA-3'
(SEQ ID NO: 57) 5'-TCCACGATTTCCCAGAGAAACA-3'

PCR primer set for mouse TNF$\alpha$ gene

(SEQ ID NO: 58) 5'-GGTGCCTATGTCTCAGCCTCTT-3'
(SEQ ID NO: 59) 5'-CGATCACCCCGAAGTTCAGTA-3'

PCR primer set for mouse Gapdh gene

(SEQ ID NO: 60) 5'-AGGTCGGTGTGAACGGATTTG-3'
(SEQ ID NO: 61) 5'-TGTAGACCATGTAGTTGAGGTCA-3

As for the expression level of each gene after normalization, the relative value of the expression level in each administration group was determined based on the expression level in the negative control group (PBS administration group) set as 1.

(3) Results

The results are shown in Fig. 8. In each group, PH-0001-hm-pr as the single-stranded nucleic acid molecule shown in SEQ ID NO: 30 showed an inhibitory effect on the expression of inflammatory cytokine genes. That is, the single-stranded nucleic acid molecule of the present invention is suggested to inhibit the onset of inflammation in uveitis via inhibition of the expression of the prorenin receptor gene.

(Example 5) Nuclease resistance and expression inhibitory effect on prorenin receptor protein

A single-stranded nucleic acid molecule shown in SEQ ID NO: 30 and having an inhibitory sequence of expression of human and mouse prorenin receptor genes was confirmed for nuclease resistance. In addition, the expression inhibitory effect on the prorenin receptor protein in human-derived cultured cells was confirmed by immunoblot analysis.

(1) Nuclease resistance

The single-stranded nucleic acid molecule shown in SEQ ID NO: 30 was incubated at 37°C in the presence of 0.5 unit of micrococcus nuclease (Takara Bio Inc.). At 5, 15 and 30 min after the start of the reaction, EDTA solution was added to the reaction solution to discontinue the reaction, electrophoresis was performed using 15% TBE gel, and the gel was stained with SYBR safe (Life Technologies). As control, a double stranded nucleic acid molecule ((P)RR-siRNA), in which the sense strand is a base sequence shown in SEQ ID NO: 19 and the antisense strand is a base sequence shown in SEQ ID NO: 8, was used.

(2) Expression inhibitory effect on prorenin receptor protein

As the cell, human retinal pigment epithelial cell (RPE, hTERT-RPE1) (ATCC) was used. As the medium, DMEM/F12 (Wako Pure Chemical Industries, Ltd.) containing 10% FBS (Life Technologies) was used. The culture conditions were 37°C, 5% $CO_2$.

The single-stranded nucleic acid molecule shown in SEQ ID NO: 30 (1nM) was transfected using a transfection reagent Lipofectamine RNAiMAX Reagent (Life Technologies) and according to the attached protocol. After transfection, the cells were cultured for 24 hr. At 3, 6, 12, 18 and 24 hr after the start of culturing, the cells were collected. Immunoblot analysis was performed as follows. The collected cells were lysed in SDS buffer containing protease inhibitor cocktail (Roche), and proteins were separated by SDS-PAGE using 10% gel and transferred to PVDF membrane (Merck Millipore). The membrane was blocked by immersing in TBS containing 5% skim milk, and reacted with a primary antibody. As the primary antibody, an anti-prorenin receptor antibody (Sigma-Aldrich), and an anti-β actin antibody (Medical & Biological Laboratories) were used. As the secondary antibody for chemiluminescent detection, an anti-mouse or anti-rabbit IgG antibody peroxidase conjugate (Jackson ImmunoResearch Laboratories) was used. For detection of the signal, enhanced chemoluminescence (Western Lightning Ultra) was used. As control, a single-stranded nucleic acid molecule having the following structural formula (Control-PshRNA) was used, wherein Lx has a structure of the aforementioned formula (I-6a).

(SEQ ID NO: 62)

5'-UACUAUUCGACACGCGAAGUUCC-Lx-GGAACUUCGCGUGUCGAAUAGUAUU-3'

(3) Results

The results of nuclease resistance are shown in Fig. 9A, and the results of the expression inhibitory effect on the prorenin receptor protein by immunoblot analysis are shown in Fig. 9B. While the control ((P)RR-siRNA) was completely degraded by micrococcus nuclease 5 min later, the single-stranded nucleic acid molecule shown in SEQ ID NO: 30 ((P)RR-PshRNA) was not degraded even after 30 min and showed nuclease resistance (Fig. 9 A). In addition, the results of the immunoblot analysis show that the single-stranded nucleic acid molecule shown in SEQ ID NO: 30 ((P)RR-PshRNA) inhibits expression of prorenin receptor protein (Fig. 9 B).

(Example 6) Acute inflammation inhibitory effect in endotoxin-induced uveitis model mouse

A single-stranded nucleic acid molecule shown in SEQ ID NO: 30 and having an inhibitory sequence of expression of human and mouse prorenin receptor genes was confirmed for an acute inflammation inhibitory effect. Using LPS-induced uveitis model mouse as an acute inflammation model mouse, the number of leukocytes adhered to retina blood vessel and the number of leukocytes infiltrating into the vitreous cavity adjacent to the optic disc were measured. In addition, to investigate the molecular mechanism by which the single-stranded nucleic acid molecule inhibits cell response, the expression levels of the inflammatory cytokine genes were measured. Furthermore, in endotoxin-induced uveitis model mouse, the inflammatory signal induces degradation of rhodopsin due to the activation of the ubiquitin proteasome system, which shortens the photoreceptor outer segment. Therefore, measurement of length of photoreceptor outer segment and immunoblot analysis of rhodopsin were performed to investigate the protective effect on photoreceptor by the single-stranded nucleic acid molecule.

(1) Administration of single-stranded nucleic acid molecule to endotoxin-induced uveitis model mouse

A single-stranded nucleic acid molecule shown in SEQ ID NO: 30 was dissolved in PBS to 100 μM. After dissolution, a single-stranded nucleic acid molecule solution (1 μL) was intravitreally administered using a 33 gauge injection needle to mice (C57BL/6J, 6- to 8-week-old) (CLEA Japan) under pentobarbital anesthesia. As control, the same amount of PBS or Control-PshRNA (SEQ ID NO: 62) dissolved in PBS to 100 μM was used. At 24 hr after administration, Escherichia coli-derived LPS (Sigma-Aldrich) (0.2 mg) dissolved in 100 μL of PBS was intraperitoneally administered to the mice administered with the single-stranded nucleic acid molecule shown in SEQ ID NO: 30 or Control-PshRNA. Using the obtained vitreous body, the expression levels of the inflammatory cytokine genes were measured at 6 hr after LPS administration. At 24 hr after LPS administration, leukocytes that adhered to the retina and leukocytes that infiltrated into the vitreous body were quantified, as well as the length of the photoreceptor outer segment was measured and immunoblot analysis with rhodopsin was performed.

(2) Measurement of inflammatory cytokine gene expression level by real-time quantitative PCR

After 6 hr from the LPS administration, the mice were euthanized by cervical dislocation, and the retina was

isolated. By the method described in a document (Kanda, A., Noda, K., Saito, W. & Ishida, S. (Pro)renin receptor is associated with angiogenic activity in proliferative diabetic retinopathy. Diabetologia 55, 3104-3113, 2012), total RNA was separated from the cells by using SuperPrep Cell Lysis & RT Kit for qPCR (TOYOBO) and from retinal tissues by using TRIzol (Life Technologies) and GoScrip Reverse Transcriptase (Promega), and cDNA was synthesized by performing a reverse transcription reaction using an oligo dT primer. Using the aforementioned synthesized cDNA as a template, real-time quantitative PCR was performed, and the expression levels of IL-6, CCL2/MCP-1, ICAM-1 and tNF-$\alpha$ genes were measured. These genes are representative genes known to be commonly involved in both acute and chronic inflammation models. In addition, the expression levels of the prorenin receptor gene and the internal standard Gapdh gene were similarly measured. The expression level of each of the aforementioned genes was normalized with reference to that of the aforementioned Gapdh gene. In the aforementioned real-time quantitative PCR, GoTaq qPCR Master Mix (Promega) was used as the reagent and StepOne plus System (Life Technologies) was used as the instrument. The same primer sets as in Example 4 were used for PCR of IL-6, CCL2/MCP-1, ICAM-1, TNF-$\alpha$ and Gapdh genes. The following primer set was used for PCR of the prorenin receptor gene. PCR primer set for prorenin gene

(SEQ ID NO: 63) 5'-CTGGTGGCGGGTGCTTTAG-3'
(SEQ ID NO: 64) 5'-GCTACGTCTGGGATTCGATCT-3'

(3) Quantification of leukocyte adhered to retina

According to the method described in a document (Kanda, A., Noda, K., Oike, Y. & Ishida, S. Angiopoietin-like protein 2 mediates endotoxin-induced acute inflammation in the eye. Lab Invest 92, 1553-1563, 2012), the retina blood vessel system and adherent leukocytes were photographed using perfusion-labeling with fluorescein isothiocyanate (FITC)-coupled concanavalin A lectin (Con A; Vector Laboratories). To be specific, 24 hr after LPS administration, the chest cavity of the mouse under anesthesia was opened and a cannula was introduced into the left ventricle. After PBS was injected to remove erythrocytes and non-adhesive leukocytes, FITC-conjugated Con A was perfused. After removal of the eyeballs, flat mount specimens of the retina were prepared. The flat mount specimens were visualized under a fluorescence microscope (BZ-9000, Keyence) and the total number of Con A-stained adherent leukocytes per retina was counted.

(4) Quantification of vitreous infiltrating leukocytes

By the method described in a document (Kanda, A., Noda, K., Oike, Y. & Ishida, S. Angiopoietin-like protein 2 mediates endotoxin-induced acute inflammation in the eye. Lab Invest 92, 1553-1563, 2012), the number of leukocytes infiltrating into the vitreous cavity was counted. To be specific, retinal tissue was fixed and embedded in paraffin by a general method. Three 5 $\mu$m-sections were prepared at 100 $\mu$m intervals. The middle section was made to pass through the optic nerve. All sections were stained with HE (Hematoxylin-Eosin) and the number of cells in the vitreous cavity was counted.

(5) Measurement of length of photoreceptor outer segment and immunoblot analysis of rhodopsin

The length of the photoreceptor outer segments were measured as follows. After fixing the eyeballs of the mouse at 4°C using 4% para-formaldehyde, they were embedded in paraffin and sections were prepared. The sections were stained with HE, and the length of the photoreceptor outer segments were measured at 4 points on the posterior retina. The two points at both ends of the optic nerve were 200 $\mu$m and 500 $\mu$m apart. The relative value of the length of the photoreceptor outer segment in the endotoxin-induced uveitis mouse administered with the single-stranded nucleic acid molecule shown in SEQ ID NO: 30 or Control-PshRNA was determined based on the length of the photoreceptor outer segment in the normal mouse (Control) set as 1. Immunoblot analysis of rhodopsin was performed by a method similar to that in Example 5 except that the retinal tissue was lysed in SDS buffer containing protease inhibitor cocktail (Roche), and an anti-rhodopsin antibody (Merck Millipore) and an anti-$\beta$ actin antibody (Medical & Biological Laboratories) were used as the primary antibody.

(6) Results

Microscopic photographs of leukocytes adhered to the retina are shown in Fig. 10 A-D (A and B: administration of control, C and D: administration of the single-stranded nucleic acid molecule shown in SEQ ID NO: 30). Arrows indicate leukocytes adhered to the retina blood vessel system with inflammation. The scale bar is 100 $\mu$m. The results of quantification of leukocytes adhered to retina are shown in Fig. 10E. The single-stranded nucleic acid molecule shown in SEQ ID NO: 30 inhibited leukocyte adhesion in retina with endotoxin-induced uveitis (Fig. 10 A-E).

Microscopic photographs of leukocytes infiltrating into the vitreous body are shown in Fig. 10F and G (F: administration of control, G: administration of the single-stranded nucleic acid molecule shown in SEQ ID NO: 30). Arrows indicate infiltrating leukocytes. The scale bar is 30 $\mu$m. The quantification results of leukocytes infiltrating into the vitreous body are shown in Fig. 10H. Leukocyte infiltration into the front of the optic disc decreased by the administration of the single-stranded nucleic acid molecule shown in SEQ ID NO: 30 (Fig. 10 F-H).

The measurement results of the expression level of the inflammatory cytokine gene are shown in Fig. 11. The expression levels of IL-6, CCL2/MCP-1, ICAM-1 and TNF-$\alpha$ genes were higher in the endotoxin-induced uveitis (EIU) mice administered with PBS or control (Control-PshRNA) than in normal mice (Control) without administration (Fig. 11 A-D). In addition, in endotoxin-induced uveitis mice administered with the single-stranded nucleic acid molecule shown in SEQ ID NO: 30 ((P)RR-PshRNA), expression of the inflammatory cytokine and prorenin receptor ((P)RR/Atp6ap2) significantly decreased (Fig. 11 A-E).

The results of the measurement of length of photoreceptor outer segment and immunoblot analysis of rhodopsin are shown in Fig. 12 (RPE is retinal pigment epithelium, OS is outer segment, IS is inner segment, ONL is outer nuclear layer, INL is inner nuclear layer). In endotoxin-induced uveitis (EIU) mice administered with the single-stranded nucleic acid molecule shown in SEQ ID NO: 30 ((P)RR-PshRNA), reduction in the length of the photoreceptor outer segment was inhibited (FIG. 12 A-D), and the decrease in rhodopsin was inhibited (Fig. 12 E).

(Example 7) Chronic inflammation inhibitory effect in streptozotocin-induced type 1 diabetes model mouse

In recent years, diabetic retinopathy is considered an inflammatory disease. Therefore, using streptozotocin-induced type 1 diabetes model mouse, chronic inflammation inhibitory effect of the single-stranded nucleic acid molecule shown in SEQ ID NO: 30 was confirmed. Measurement of the expression levels of inflammatory cytokine genes and quantification of leukocytes adhered to the retina blood vessel were performed.

(1) Administration of single-stranded nucleic acid molecule to streptozotocin-induced type 1 diabetes model mouse

Streptozotocin (Sigma) (60 mg/kg body weight) dissolved in citric acid solution was consecutively administered for 4 days by intraperitoneal injection to mice (C57BL/6J, 6- to 8-week-old) (CLEA Japan). The mice with plasmaglucose concentration exceeding 250 mg/dl on day 7 after Streptozotocin administration was taken as diabetic and used for the subsequent experiment. After 2 months from Streptozotocin administration, the single-stranded nucleic acid molecule shown in SEQ ID NO: 30 was dissolved in PBS to 100 $\mu$M, and the solution (1 $\mu$L) was vitreously administered using a 33 gauge injection needle to the mouse under pentobarbital anesthesia. As control, the same amount of PBS or Control-PshRNA (SEQ ID NO: 62) dissolved in PBS to 100 $\mu$M was used. At 24 hr from the administration, the expression levels of the inflammatory cytokine genes were measured. In addition, leukocytes that adhered to the retina at 48 hr after administration were quantified.

(2) Measurement of inflammatory cytokine gene expression level by real-time quantitative PCR

By a method similar to that in Example 6, the expression levels of IL-6, CCL2/MCP-1, ICAM-1, TNF-$\alpha$ and Gapdh genes were measured.

(3) Quantification of leukocytes adhered to retina

By a method similar to that in Example 6, the leukocytes that adhered to the retina were quantified.

(4) Results

Microscopic photographs of leukocytes adhered to the retina are shown in Fig. 13 A-D (A and B: administration of control, C and D: administration of the single-stranded nucleic acid molecule shown in SEQ ID NO: 30). The scale bar is 100 $\mu$m. The results of quantification of leukocytes adhered to retina are shown in Fig. 13E. Administration of the single-stranded nucleic acid molecule shown in SEQ ID NO: 30 significantly decreased the number of leucocytes (Fig. 13 A-E).

The measurement results of the expression level of the inflammatory cytokine gene are shown in Fig. 13 F-I (Control is expression level of normal mouse without administration of PBS or nucleic acid). The expression of IL-6, CCL2/MCP-1, ICAM-1, and TNF-$\alpha$ genes in the diabetic mouse administered with the single-stranded nucleic acid molecule shown in SEQ ID NO: 30 ((P)RR-PshRNA) was significantly inhibited as compared to that in diabetic mouse administered with the PBS or control (Control-PshRNA).

(Example 8) Angiogenesis inhibitory effect in laser-induced CNV model mouse

An angiogenesis inhibitory effect of the single-stranded nucleic acid molecule shown in SEQ ID NO: 30 and having an inhibitory sequence of expression of human and mouse prorenin receptor genes was confirmed in laser-induced CNV model mouse.

(1) Administration of single-stranded nucleic acid molecule to laser-induced CNV (choroidal neovascularization) model mouse

C57BL/6J mice (male, 6- to 8-week-old) (CLEA Japan) were anesthetized by intraperitoneal injection of pentobarbital (0.05 mg/g body weight), and the pupils were dilated with 5% phenylephrine hydrochloride and 5% tropicamide. Laser photocoagulation was performed with a slit lamp delivery system using a cover glass as a contact lens by using ND:YAG 532-mn laser (Novus Spectra; Lumenis). Each eye was irradiated with four laser spots surrounding the optic nerve (180 mW, 75 μm, 100 ms) and CNV (choroidal neovascularization) model mice were created. Immediately after laser photocoagulation, a single-stranded nucleic acid molecule solution (1 μL) obtained by dissolution in PBS to 100 μM was intravitreally administered using a 33 gauge injection needle. As control, the same amount of PBS or Control-PshRNA (SEQ ID NO: 62) dissolved in PBS to 100 μM was used. Rupture of Bruch membrane was confirmed by the appearance of air bubbles during laser injury. Eyes with vitreous bleeding, retinal bleeding or subretinal bleeding were excluded from this test.

(2) Quantification of laser-induced CNV

Mice were euthanized by overdose of anesthesia 7 days after laser photocoagulation, and eyeballs were removed and fixed with 4% para-formaldehyde for 1 hr. After removal of the anterior ocular segment and neural retina, four sites were radially incised to flatten the retinal pigment epithelium-choroid complex to prepare the choroidal flat mounts. The choroidal flat mounts were immersed in PBS containing 5% goat serum and 1% Triton X-100 and incubated at room temperature for 1 hr, after which incubated together with fluorescence-labeled isolectin-B4 at 4°C overnight. The choroidal flat mounts were observed under a fluorescence microscope (Biorevo, Keyence), and the area of CNV was measured using a microscope software (BZ-II analyzer).

(3) Results

The measurement results of CNV area are shown in Fig. 14A, and the microscopic photographs of CNV are shown in Fig. 14B-D. The CNV area in the retina administered with the single-stranded nucleic acid molecule shown in SEQ ID NO: 30 ((P)RR-PshRNA) was significantly small as compared to that with PBS or control (Control-PshRNA).

While the present invention has been described above with reference to illustrative embodiments, the present invention is by no means limited thereto. Various changes that may become apparent to those skilled in the art may be made in the configuration and specifics of the present invention without departing from the scope of the present invention.

The contents disclosed in any publication cited herein, including patents and patent applications, are hereby incorporated in their entireties by reference, to the extent that they have been disclosed herein.

[Industrial Applicability]

According to the single-stranded nucleic acid molecule of the present invention, expression of prorenin gene or prorenin receptor gene can be inhibited. Therefore, the present invention is effective as a therapeutic agent for diseases caused by the expression of prorenin gene or prorenin receptor gene, for example, uveitis, diabetic retinopathy and the like.

This application is based on a patent application No. 2015-257713 filed in Japan (filing date: December 29, 2015), the contents of which are incorporated in full herein.

## Claims

1. A single-stranded nucleic acid molecule inhibiting expression of a prorenin gene or a prorenin receptor gene, comprising only region (X), linker region (Lx) and region (Xc), wherein the aforementioned linker region (Lx) has a non-nucleotide structure comprising at least one of a pyrrolidine skeleton and a piperidine skeleton, and
one of the aforementioned region (X) and the aforementioned region (Xc) comprises an expression inhibitory sequence comprising a nucleotide sequence consisting of at least 18 continuous nucleotides in any nucleotide sequence selected from a sequence complementary to a part of a prorenin gene represented by the following SEQ ID NO: 1 to 5:

(SEQ ID NO: 1) 5'-UGAUGUUGUCGAAGAUAGGGG-3'
(SEQ ID NO: 2) 5'-UUGAUAUAGUGGAAAUUCCCU-3'
(SEQ ID NO: 3) 5'-UGAUAUAGUGGAAAUUCCCUU-3'
(SEQ ID NO: 4) 5'-UAGAACUUUCGGAUGAAGGUG-3'
(SEQ ID NO: 5) 5'-AUCAAACUCUGUGUAGAACUU-3'

and a sequence complementary to a part of a prorenin receptor gene represented by the following SEQ ID NO: 6 to 11:

(SEQ ID NO: 6) 5'-UUCCAUUUCGGAAAACAACAG-3'
(SEQ ID NO: 7) 5'-AAUUUCCAUUUCGGAAAACAA-3'
(SEQ ID NO: 8) 5'-UUAUAUGCAAGGUUAUAGGGA-3'
(SEQ ID NO: 9) 5'-UAUAUGCAAGGUUAUAGGGAC-3'
(SEQ ID NO: 10) 5'-AAAAGGAACUGCAUUCUCCAA-3'
(SEQ ID NO: 11) 5'-UUAGUAUCCAUAUUAGCCCAU-3', and

the other comprises a nucleotide sequence complementary to the expression inhibitory sequence.

2. The nucleic acid molecule according to claim 1, wherein the region (X), the linker region (Lx) and the region (Xc) are disposed in this order from the 3'-side to the 5'-side, and a base number (X) of the aforementioned region (X)

and a base number (Xc) of the aforementioned region (Xc) satisfy the conditions of the following formula (1) or the formula (2):

$$X>Xc... \quad (1)$$

$$X=Xc... \quad (2).$$

3. The nucleic acid molecule according to claim 2, wherein the base number (X) of the aforementioned region (X) and the base number (Xc) of the aforementioned region (Xc) satisfy the conditions of the following formula (3):

$$X-Xc=1,\ 2\ or\ 3... \quad (3).$$

4. The nucleic acid molecule according to any one of claims 1 to 3, wherein the base number (Xc) of the aforementioned region (Xc) is 19 - 30.

5. The nucleic acid molecule according to any one of claims 1 to 4, wherein the aforementioned linker region (Lx) is represented by the following formula (I):

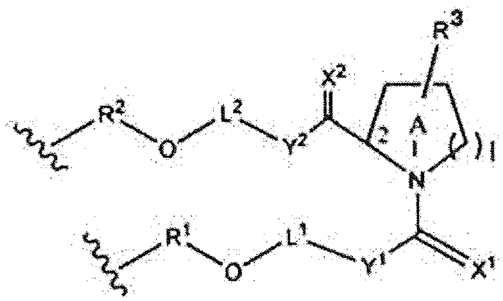

... (I)

wherein,

$X^1$ and $X^2$ are each independently $H_2$, O, S, or NH;
$Y^1$ and $Y^2$ are each independently a single bond, $CH_2$, NH, O, or S;
$R^3$ is a hydrogen atom or a substituent bonded to C-3, C-4, C-5 or C-6 on ring A,
$L^1$ is an alkylene chain composed of n atoms, and a hydrogen atom on an alkylene carbon atom may or may not be substituted with OH, $OR^a$, $NH_2$, $NHR^a$, $NR^aR^b$, SH, or $SR^a$, or
$L^1$ is a polyether chain obtained by substituting at least one carbon atom on the aforementioned alkylene chain with an oxygen atom,
provided that: when $Y^1$ is NH, O, or S, an atom bound to $Y^1$ in $L^1$ is carbon, an atom bound to $OR^1$ in $L^1$ is carbon, and oxygen atoms are not adjacent to each other;
$L^2$ is an alkylene chain composed of m atoms, and a hydrogen atom on an alkylene carbon atom may or may not be substituted with OH, $OR^c$, $NH_2$, $NHR^c$, $NR^cR^d$, SH, or $SR^c$, or
$L^2$ is a polyether chain obtained by substituting at least one carbon atom on the aforementioned alkylene chain with an oxygen atom,
provided that: when $Y^2$ is NH, O, or S, an atom bound to $Y^2$ in $L^2$ is carbon, an atom bound to $OR^2$ in $L^2$ is carbon, and oxygen atoms are not adjacent to each other;
$R^a$, $R^b$, $R^c$, and $R^d$ are each independently a substituent or a protecting group;
1 is 1 or 2;
m is an integer in the range from 0 to 30;
n is an integer in the range from 0 to 30;
in ring A, one carbon atom other than C-2 on the aforementioned ring A may be substituted by nitrogen, oxygen or sulfur,
the aforementioned ring A may contain a carbon-carbon double bond or a carbon-nitrogen double bond,

the aforementioned regions (Xc) and (X) are each linked to the aforementioned linker region (Lx) via $-OR^1-$ or $-OR^2-$,

wherein $R^1$ and $R^2$ may or may not be present, and when they are present, $R^1$ and $R^2$ are each independently a nucleotide residue or the aforementioned structure (I).

**6.** The nucleic acid molecule according to any one of claims 1 to 5, wherein the aforementioned linker region (Lx) is represented by the following formula (I-4a) or (I-6a):

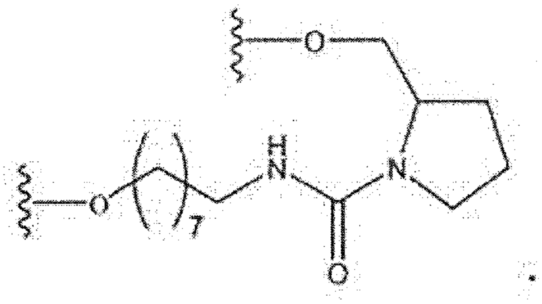

• • • (I-4a)

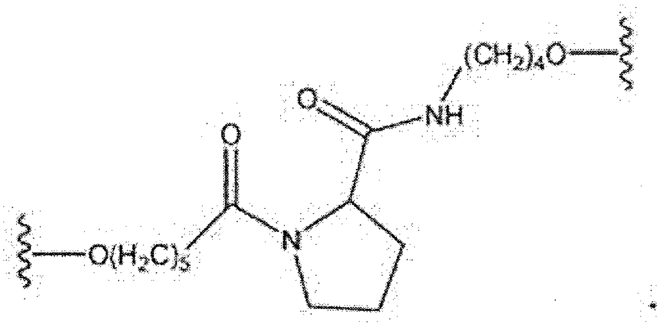

• • • (I-6a)

**7.** The nucleic acid molecule according to any one of claims 1 to 6, which is an RNA molecule.

**8.** The nucleic acid molecule according to any one of claims 1 to 7, comprising at least one modified residue.

**9.** The nucleic acid molecule according to any one of claims 1 to 8, comprising a labeling substance.

**10.** The nucleic acid molecule according to any one of claims 1 to 9, comprising a stable isotope.

**11.** The nucleic acid molecule according to any one of claims 1 to 10, wherein a total of the base number is not less than 38.

**12.** The nucleic acid molecule according to any one of claims 1 to 11, composed of a base sequence represented by any of the following SEQ ID NO: 23 to 33:

(SEQ ID NO: 23)
5'-CCUAUCUUCGACAACAUCAUCCC-Lx-GGGAUGAUGUUGUCGAAGAUAGGGG-3'
(SEQ ID NO: 24)
5'-GGAAUUUCCACUAUAUCAACCCC-Lx-GGGGUUGAUAUAGUGGAAAUUCCCU-3'
(SEQ ID NO: 25)
5'-GGGAAUUUCCACUAUAUCAACCC-Lx-GGGUUGAUAUAGUGGAAAUUCCCUU-3'
(SEQ ID NO: 26)
5'-CCUUCAUCCGAAAGUUCUACACC-Lx-GGUGUAGAACUUUCGGAUGAAGGUG-3'
(SEQ ID NO: 27)
5'- GUUCUACACAGAGUUUGAUCGCC-Lx-GGCGAUCAAACUCUGUGUAGAACUU-3'
(SEQ ID NO: 28)
5'-GUUGUUUUCCGAAAUGGAAAUCC-Lx-GGAUUUCCAUUUCGGAAAACAACAG-3'
(SEQ ID NO: 29)
5'-GUUUUCCGAAAUGGAAAUUGGCC-Lx-GGCCAAUUUCCAUUUCGGAAAACAA-3'

(SEQ ID NO: 30)
5'-CCUAUAACCUUGCAUAUAAGUCC-Lx-GGACUUAUAUGCAAGGUUAUAGGGA-3'
(SEQ ID NO: 31)
5'-CCCUAUAACCUUGCAUAUAAGCC-Lx-GGCUUAUAUGCAAGGUUAUAGGGAC-3'
(SEQ ID NO: 32)
5'-GGAGAAUGCAGUUCCUUUUAGCC-Lx-GGCUAAAAGGAACUGCAUUCUCCAA-3'
(SEQ ID NO: 33)
5'-GGGCUAAUAUGGAUACUAAAACC-Lx-GGUUUUAGUAUCCAUAUUAGCCCAU-3'

**13.** An inhibitor of expression of a prorenin gene or a prorenin receptor gene, comprising the nucleic acid molecule according to any one of claims 1 to 12.

**14.** A medicament comprising the nucleic acid molecule according to any one of claims 1 to 12.

**15.** A therapeutic agent for an eye disease, comprising the nucleic acid molecule according to any one of claims 1 to 12.

**16.** The agent according to claim 15, wherein the eye disease is selected from the group consisting of diabetic retinopathy, uveitis, age-related macular degeneration and glaucoma.

**17.** A method for inhibiting expression of a prorenin gene or a prorenin receptor gene, comprising using the nucleic acid molecule according to any one of claims 1 to 12.

**18.** The method according to claim 17, comprising a step of administering the aforementioned nucleic acid molecule to a cell, a tissue or an organ.

**19.** The method according to claim 18, wherein the aforementioned administration is performed in vitro.

**20.** The nucleic acid molecule according to any one of claims 1 to 12 for use in inhibiting expression of a prorenin gene or a prorenin receptor gene.

**21.** Use of the nucleic acid molecule according to any one of claims 1 to 12 for the production of an inhibitor of expression of a prorenin gene or a prorenin receptor gene.

Fig. 1

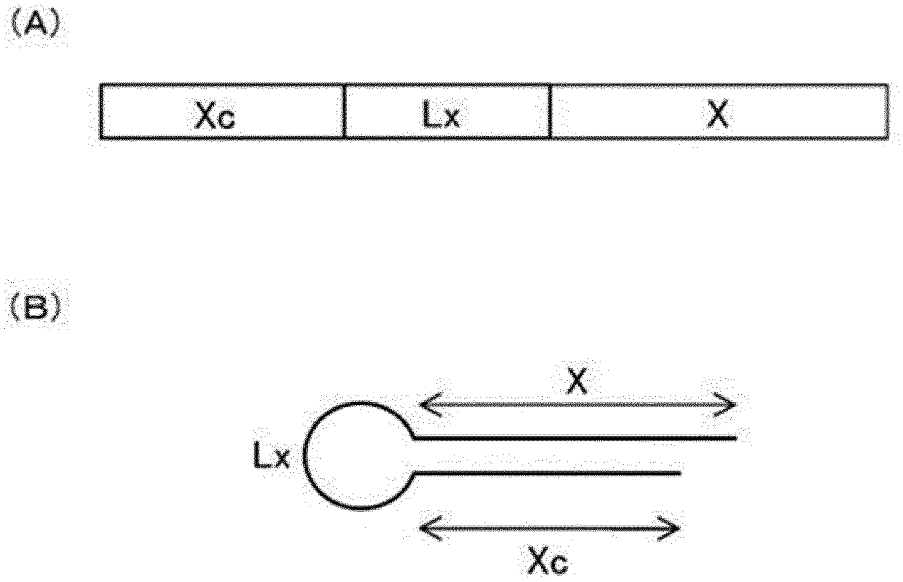
(A)
Xc
Lx
X
(B)
X
Lx
Xc

Fig. 2

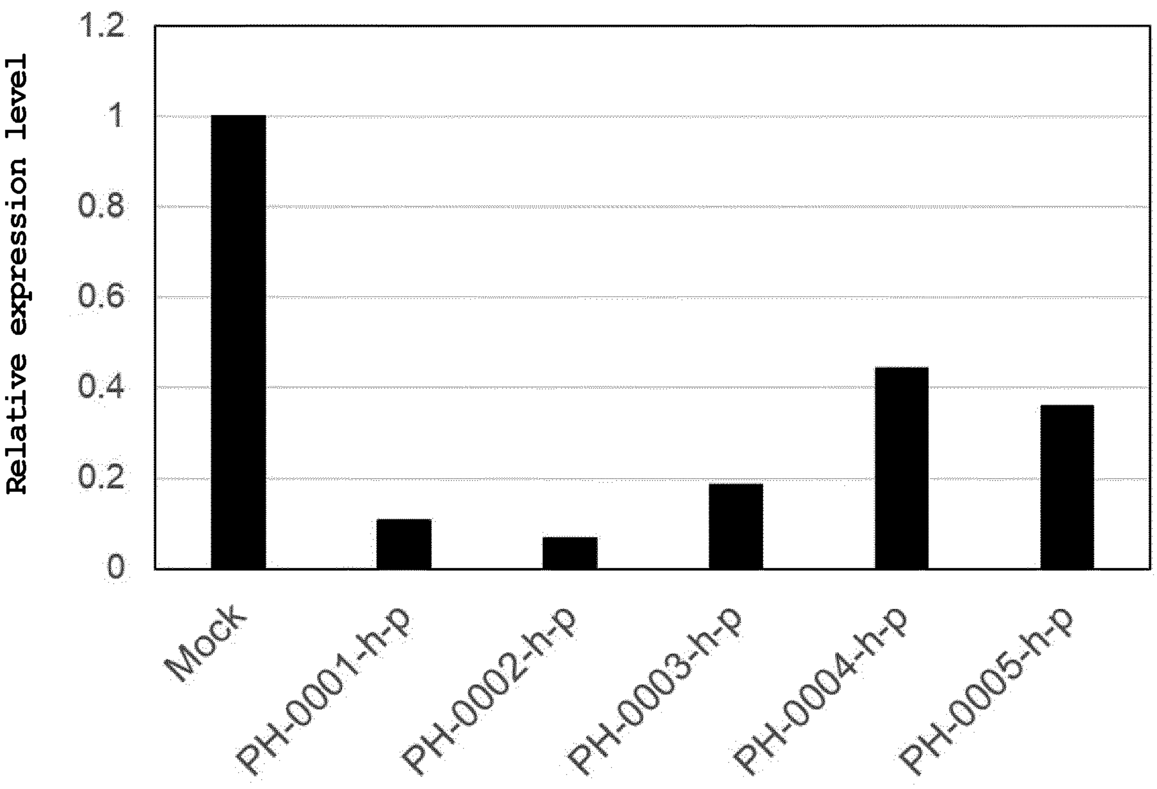
Relative expression level
1.2
1
0.8
0.6
0.4
0.2
0
Mock
PH-0001-h-p
PH-0002-h-p
PH-0003-h-p
PH-0004-h-p
PH-0005-h-p

Fig. 3

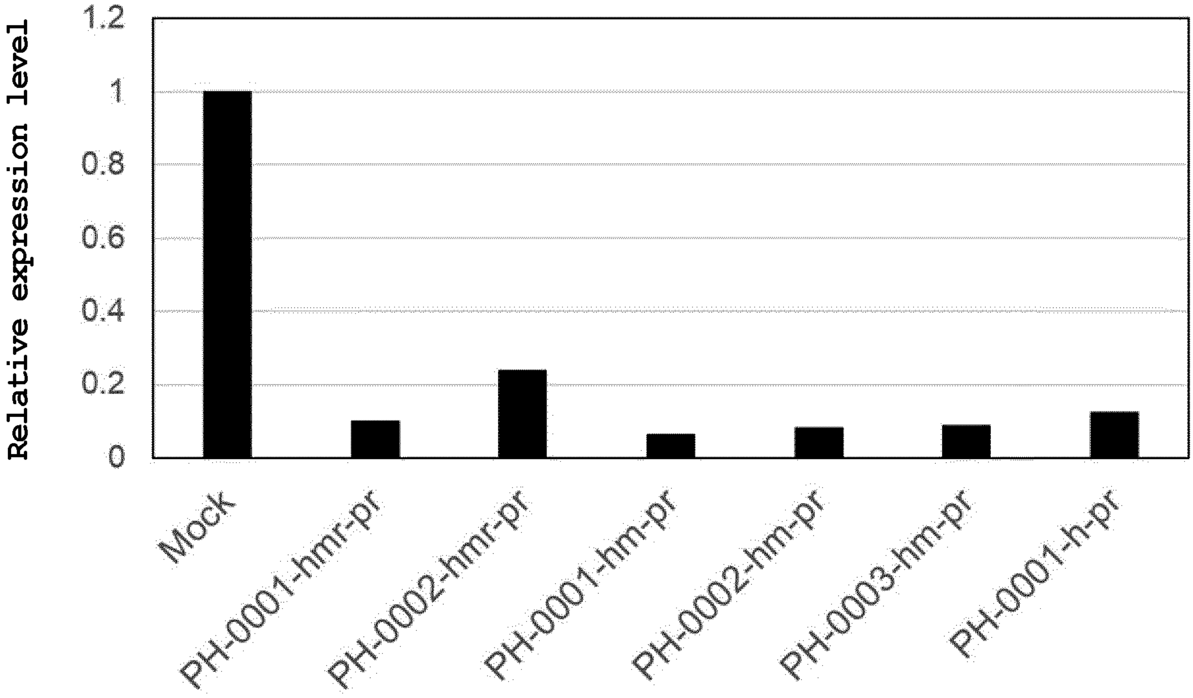
Relative expression level
1.2
1
0.8
0.6
0.4
0.2
0
Mock
PH-0001-hmr-pr
PH-0002-hmr-pr
PH-0001-hm-pr
PH-0002-hm-pr
PH-0003-hm-pr
PH-0001-h-pr

Fig. 4

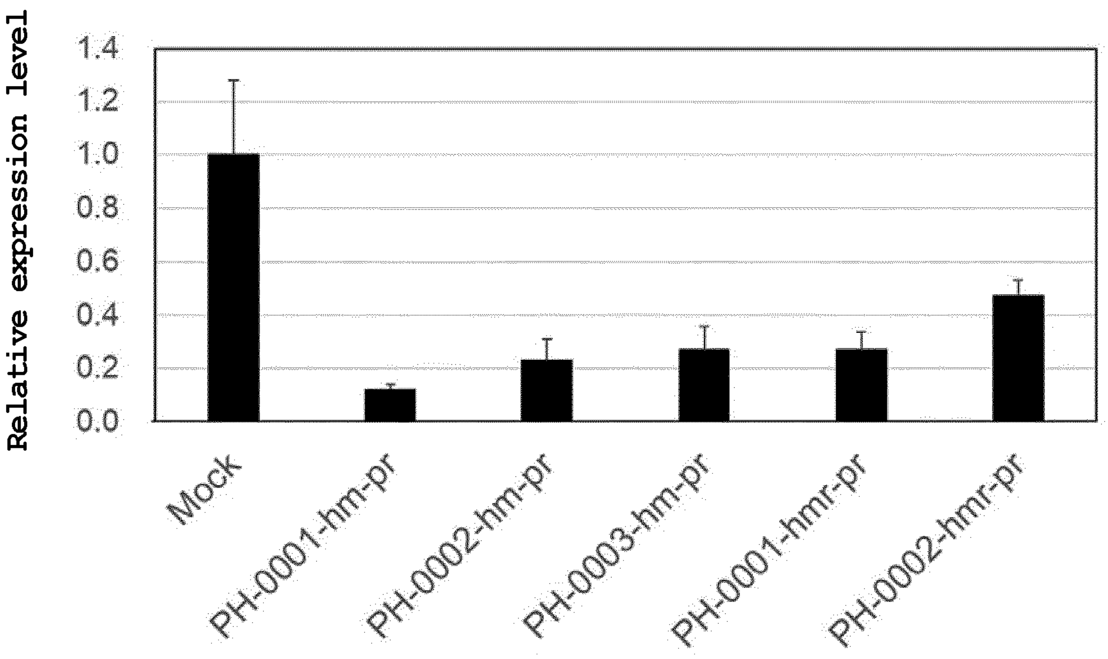
Relative expression level
1.4
1.2
1.0
0.8
0.6
0.4
0.2
0.0
Mock
PH-0001-hm-pr
PH-0002-hm-pr
PH-0003-hm-pr
PH-0001-hmr-pr
PH-0002-hmr-pr

Fig. 5

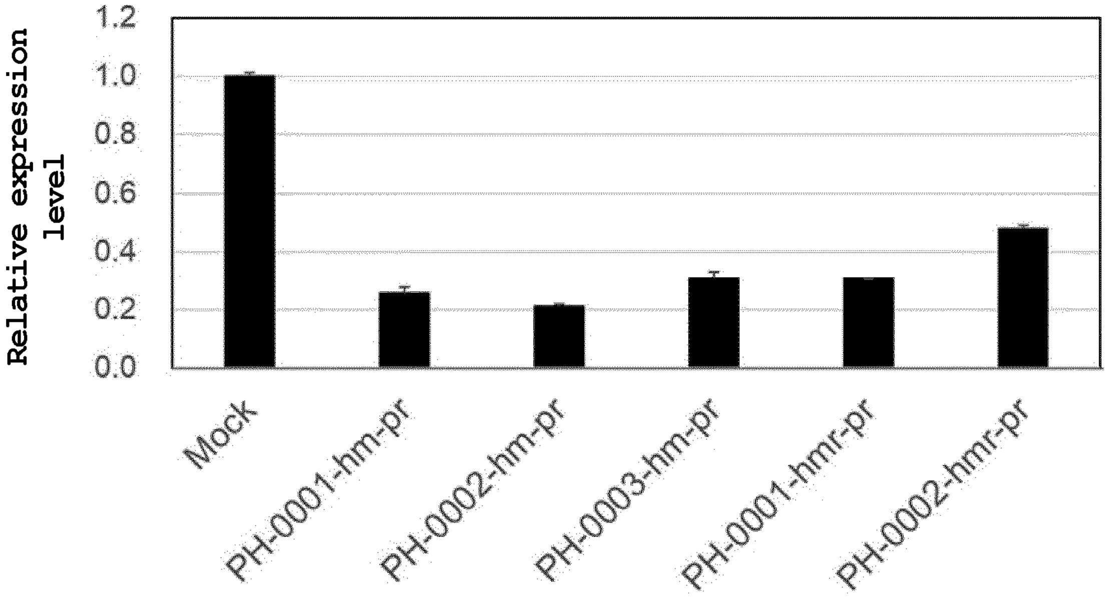
Relative expression level
1.2
1.0
0.8
0.6
0.4
0.2
0.0
Mock
PH-0001-hm-pr
PH-0002-hm-pr
PH-0003-hm-pr
PH-0001-hmr-pr
PH-0002-hmr-pr

Fig. 6

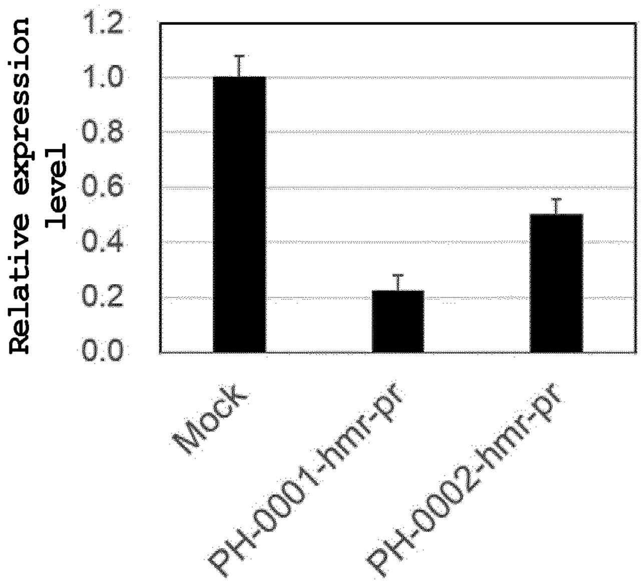
Relative expression level
1.2
1.0
0.8
0.6
0.4
0.2
0.0
Mock
PH-0001-hmr-pr
PH-0002-hmr-pr

Fig. 7

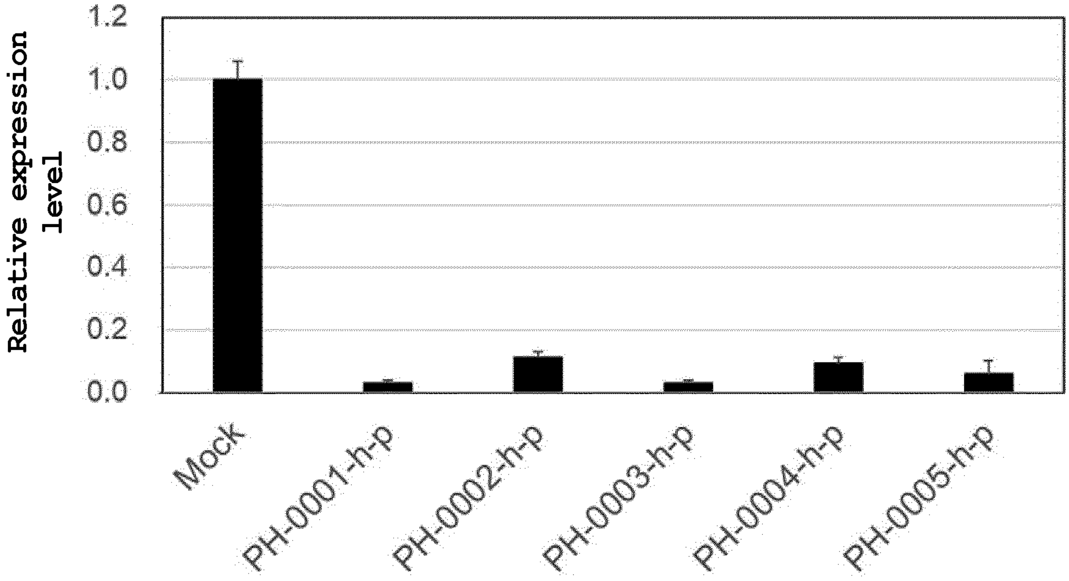
Relative expression level
1.2
1.0
0.8
0.6
0.4
0.2
0.0
Mock
PH-0001-h-p
PH-0002-h-p
PH-0003-h-p
PH-0004-h-p
PH-0005-h-p

Fig. 8

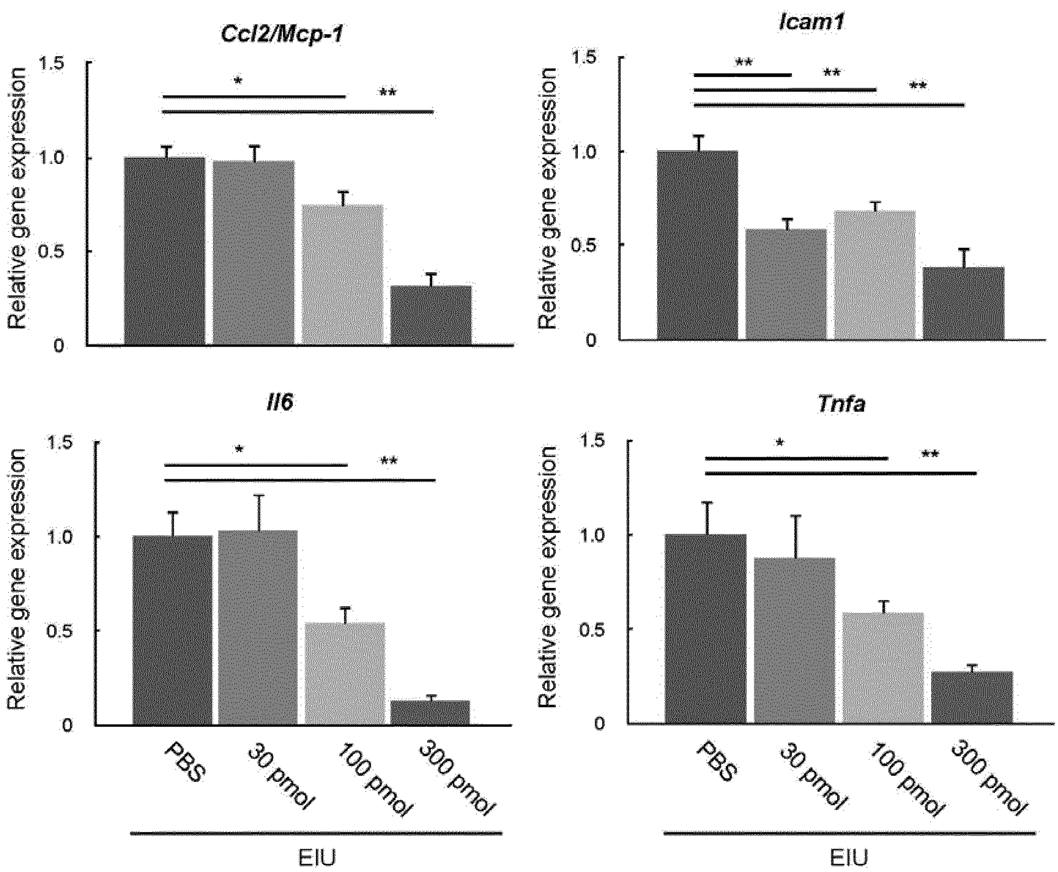
Ccl2/Mcp-1
Icam1
Il6
Tnfa
Relative gene expression
1.5
1.0
0.5
0
*
**
PBS
30 pmol
100 pmol
300 pmol
EIU

Fig. 9

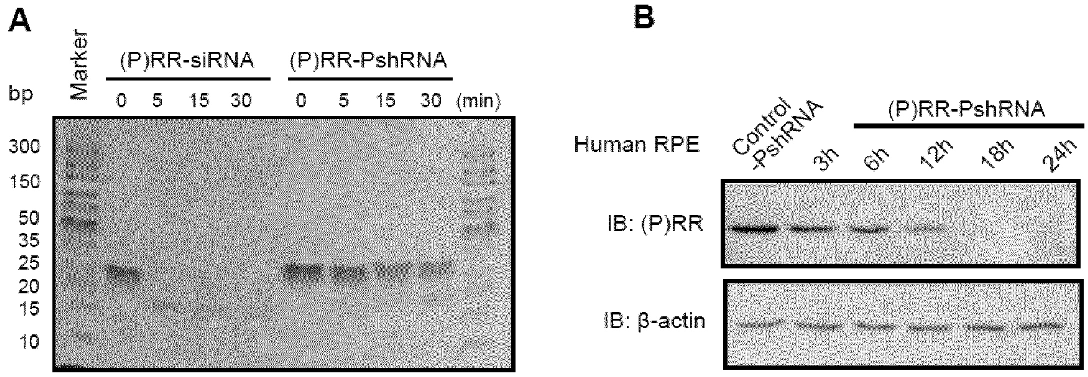
A
Marker
(P)RR-siRNA
(P)RR-PshRNA
bp
0 5 15 30 0 5 15 30 (min)
300
150
50
35
25
20
15
10
B
Human RPE
Control -PshRNA
(P)RR-PshRNA
3h
6h
12h
18h
24h
IB: (P)RR
IB: β-actin

Fig. 10

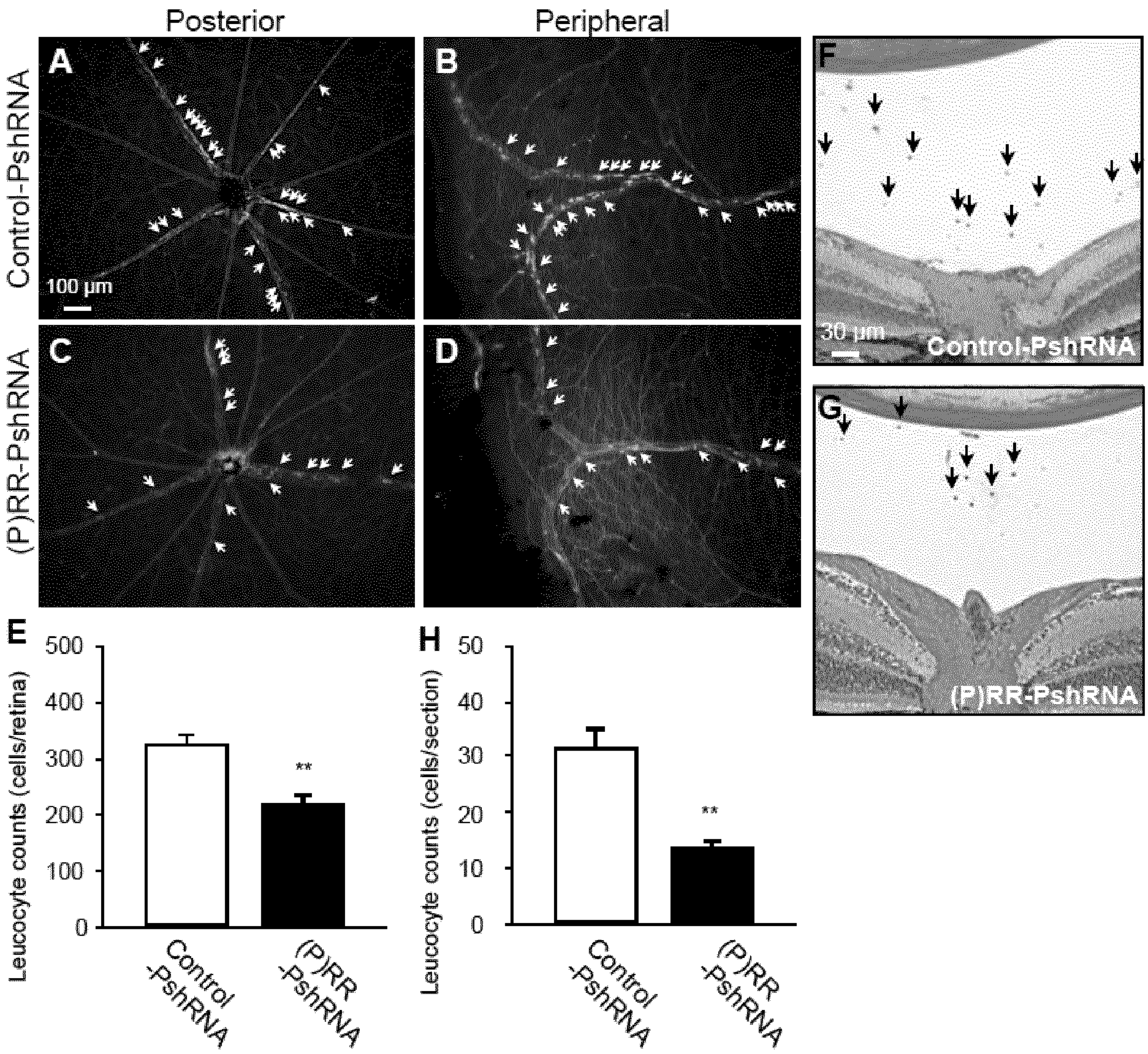
Posterior
Peripheral
Control-PshRNA
(P)RR-PshRNA
A
B
C
D
100 μm
F
30 μm
Control-PshRNA
G
(P)RR-PshRNA
E
Leucocyte counts (cells/retina)
500
400
300
200
100
0
**
Control -PshRNA
(P)RR -PshRNA
H
Leucocyte counts (cells/section)
50
40
30
20
10
0
**
Control -PshRNA
(P)RR -PshRNA

Fig. 11

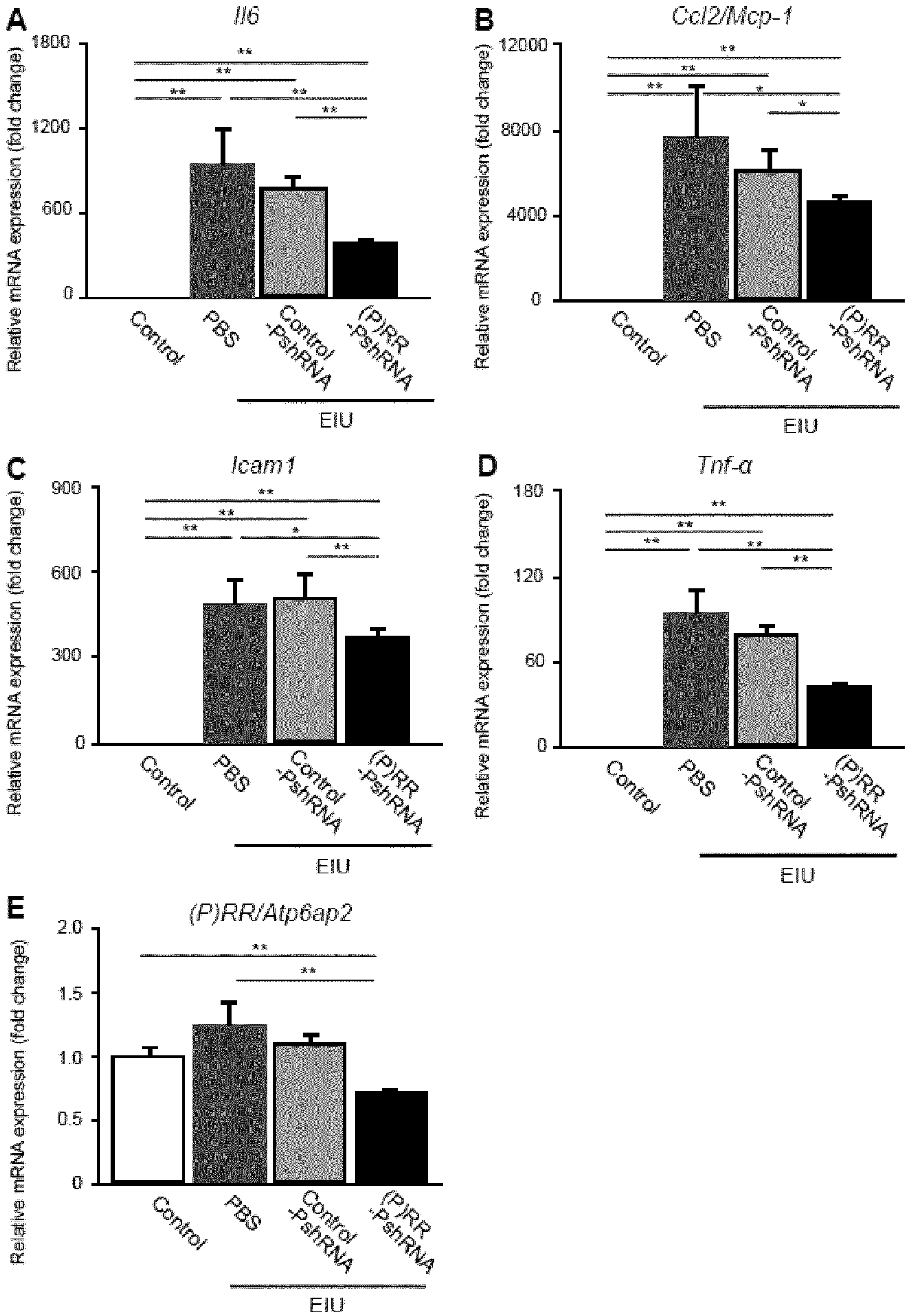
A
Il6
Relative mRNA expression (fold change)
1800
1200
600
0
**
*
Control
PBS
Control -PshRNA
(P)RR -PshRNA
EIU
B
Ccl2/Mcp-1
12000
8000
4000
0
C
Icam1
900
600
300
0
D
Tnf-α
180
120
60
0
E
(P)RR/Atp6ap2
2.0
1.5
1.0
0.5
0

Fig. 12

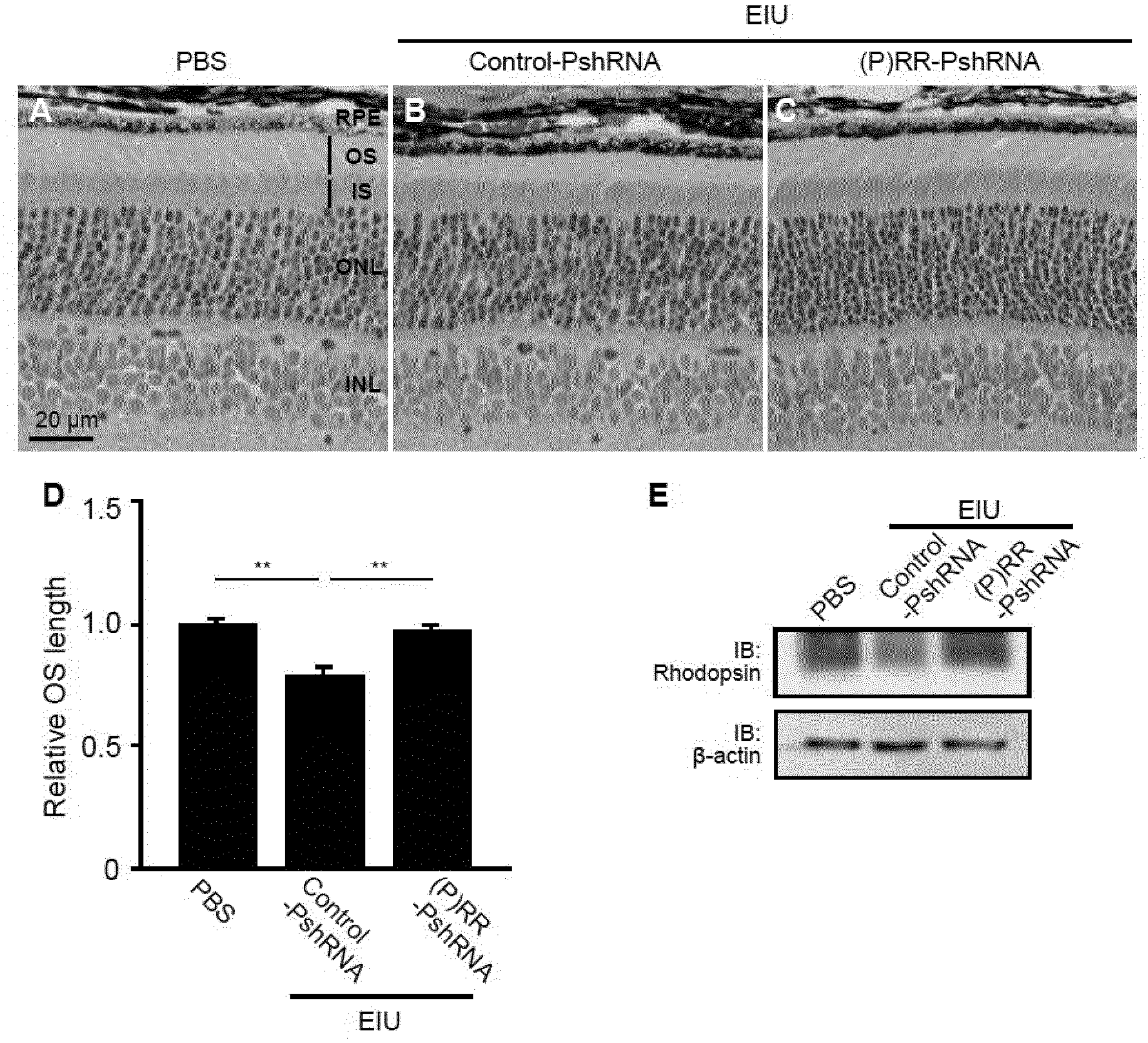
EIU
PBS
Control-PshRNA
(P)RR-PshRNA
A
B
C
RPE
OS
IS
ONL
INL
20 μm
D
Relative OS length
1.5
1.0
0.5
0
**
**
PBS
Control
-PshRNA
(P)RR
-PshRNA
EIU
E
EIU
PBS
Control
-PshRNA
(P)RR
-PshRNA
IB:
Rhodopsin
IB:
β-actin

Fig. 13

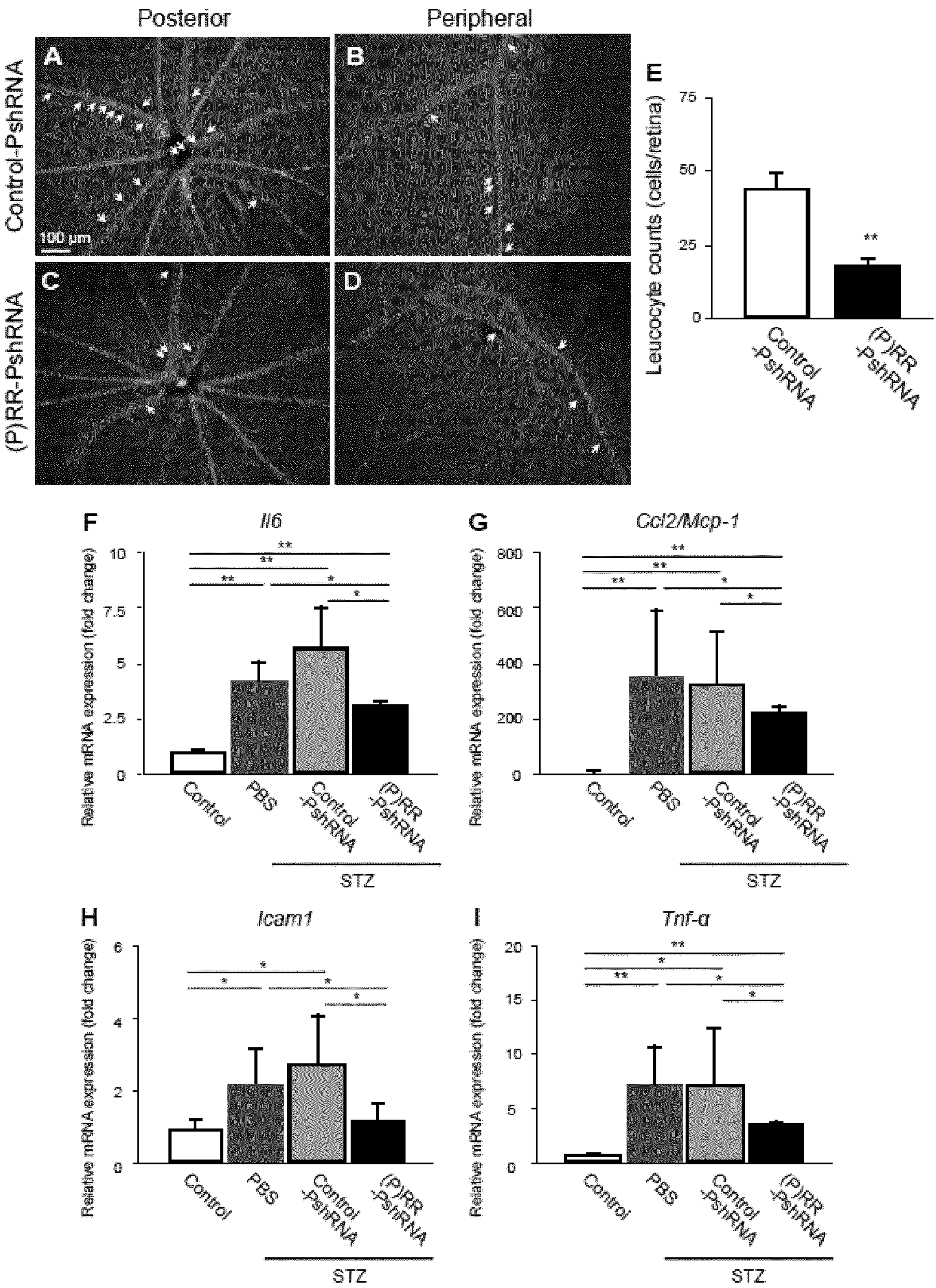
Posterior
Peripheral
Control-PshRNA
(P)RR-PshRNA
A
B
C
D
100 µm
E
Leucocyte counts (cells/retina)
75
50
25
0
**
Control -PshRNA
(P)RR -PshRNA
F
Il6
Relative mRNA expression (fold change)
10
7.5
5
2.5
0
Control
PBS
Control -PshRNA
(P)RR -PshRNA
STZ
G
Ccl2/Mcp-1
800
600
400
200
0
H
Icam1
6
4
2
0
I
Tnf-α
20
15
10
5
0

Fig. 14

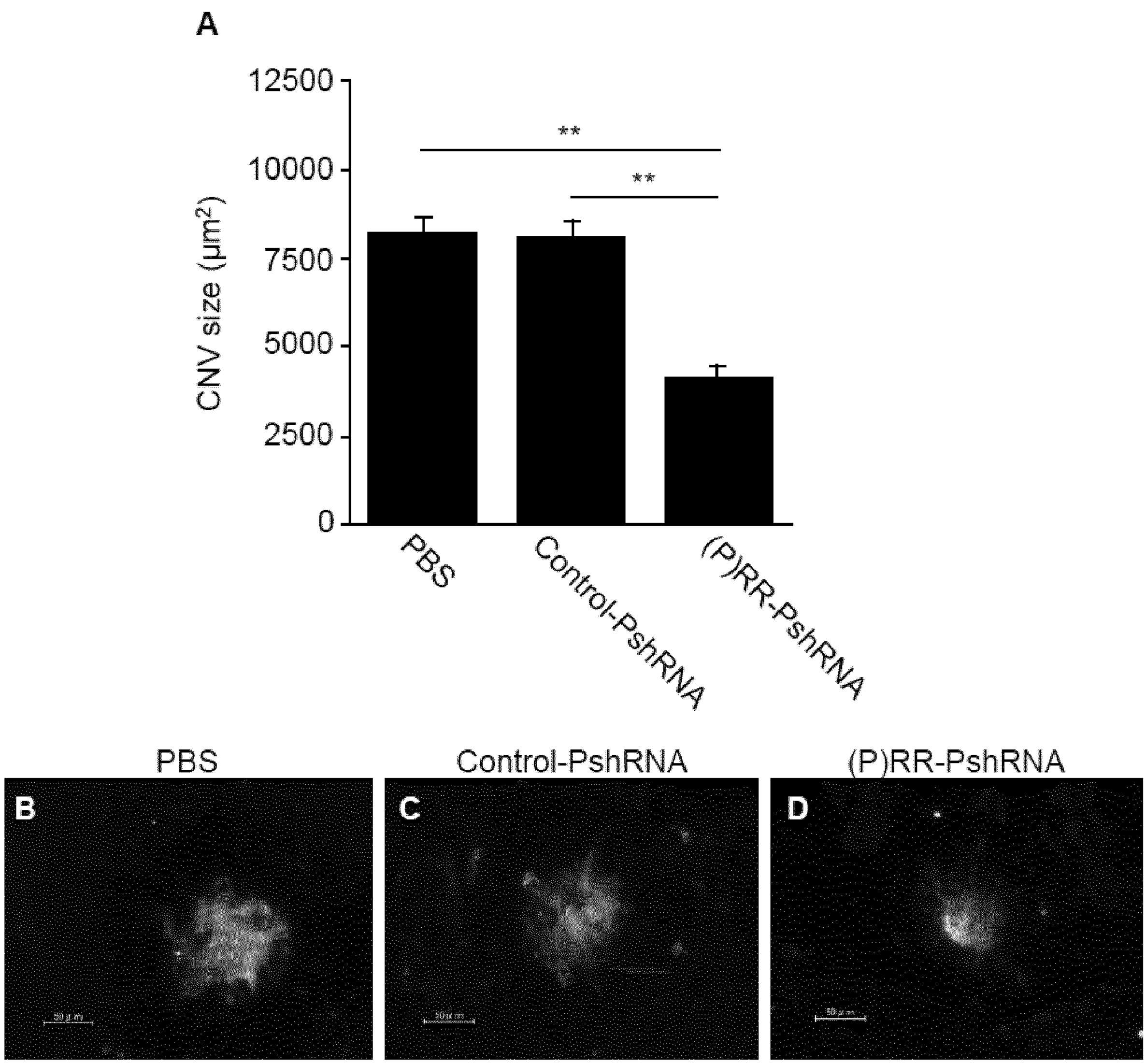
A
12500
10000
7500
5000
2500
0
CNV size (μm²)
**
**
PBS
Control-PshRNA
(P)RR-PshRNA
PBS
Control-PshRNA
(P)RR-PshRNA
B
C
D

## INTERNATIONAL SEARCH REPORT

International application No.
PCT/JP2016/089216

A. CLASSIFICATION OF SUBJECT MATTER
*C12N15/113*(2010.01)i, *A61K31/7088*(2006.01)i, *A61K31/7105*(2006.01)i, *A61K48/00*(2006.01)i, *A61P27/02*(2006.01)i

According to International Patent Classification (IPC) or to both national classification and IPC

B. FIELDS SEARCHED

Minimum documentation searched (classification system followed by classification symbols)
C12N15/113, A61K31/7088, A61K31/7105, A61K48/00, A61P27/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/MEDLINE/EMBASE/BIOSIS(STN), JSTPlus/JMEDPlus/JST7580(JDreamIII), GenBank/EMBL/DDBJ/GeneSeq, PubMed

C. DOCUMENTS CONSIDERED TO BE RELEVANT

| Category* | Citation of document, with indication, where appropriate, of the relevant passages | Relevant to claim No. |
|---|---|---|
| Y | BATENBURG, W.W., et al., Combined Renin Inhibition/(Pro)Renin Receptor Blockade in Diabetic Retinopathy- A Study in Transgenic (mREN2)27 Rats, 2014, PLOS ONE, Vol.9, No.6, e100954, p.1-11, fig. 6, page 5, right column, 1st paragraph, page 10, left column, 2nd paragraph to right column, 2nd paragraph, abstract | 1-21 |
| Y | JP 2008-510786 A (Sylentis S.A.U.), 10 April 2008 (10.04.2008), claim 35; SEQ ID NO:1140 to 1196; claims 3 to 5 & WO 2006/021817 A2 claim 35; SEQ ID NO:1140 to 1196; claims 3 to 5 & US 2007/0270365 A1 & EP 1781787 A1 & EP 2292757 A2 & EP 2298892 A2 | 1-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 March 2017 (15.03.17) | 28 March 2017 (28.03.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2016/089216

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2015/093495 A1 (Bonac Corp. et al.), 25 June 2015 (25.06.2015), claim 1; example 1 (Family: none) | 1-21 |
| Y | WO 2012/017919 A1 (Bonac Corp.), 09 February 2012 (09.02.2012), claim 1; example B1 (Family: none) | 1-21 |
| Y | JP 2013-055913 A (Bonac Corp.), 28 March 2013 (28.03.2013), claim 4; example 1 (Family: none) | 1-21 |
| Y | SATOFUKA, S., et al., Suppression of Ocular Inflammation in Endotoxin-Induced Uveitis by Inhibiting Nonproteolytic Activation of Prorenin, 2006, Investigative Ophthalmology and Visual Science, Vol.47, No.6, p.2686-2692, page 2686, left column, 3rd paragraph to right column, 1st paragraph | 1-21 |
| A | HAMASAKI, T., et al., Efficacy of a Novel Class of RNA Interference Therapeutic Agents, 2012, PLOS ONE, Vol.7, No.8, e42655, p.1-15 entire text | 1-21 |
| A | Atsuhiro KANDA, "(Pro)renin Receptor in the Pathogenesis of Proliferative Diabetic Retinopathy", 2014, vol.118, no.11, pages 916 to 926, entire text | 1-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012005368 A
- WO 2012017919 A
- US 3687808 A
- US 46566503 P
- US 0407070 W
- WO 2013027843 A
- JP 2015257713 A


### Non-patent literature cited in the description


- **HAMASAKI et al.** *PLoS ONE,* vol. 7 (8), e42655
- **LIMBACH et al.** Summary: the modified nucleosides of RNA. *Nucleic Acids Res.,* 1994, vol. 22, 2183-2196
- Concise Encyclopedia of Polymer Science and Engineering. John Wiley & Sons, 1990, 858-859
- **ENGLISCH et al.** *Angewandte Chemie, International Edition,* 1991, vol. 30, 613
- **KANDA, A. ; NODA, K. ; SAITO, W. ; ISHIDA, S.** Pro)renin receptor is associated with angiogenic activity in proliferative diabetic retinopathy. *Diabetologia,* 2012, vol. 55, 3104-3113
- **KANDA, A. ; NODA, K. ; OIKE, Y. ; ISHIDA, S.** Angiopoietin-like protein 2 mediates endotoxin-induced acute inflammation in the eye. *Lab Invest,* 2012, vol. 92, 1553-1563